(19) Europäisches Patentamt / European Patent Office / Office européen des brevets

(11) **EP 3 187 116 B1**

(12) **EUROPEAN PATENT SPECIFICATION**

(45) Date of publication and mention
of the grant of the patent:
**23.12.2020 Bulletin 2020/52**

(51) Int Cl.:
*A61B 10/00* $^{(2006.01)}$    *A61B 5/0456* $^{(2006.01)}$
*A61B 5/11* $^{(2006.01)}$

(21) Application number: **15836914.0**

(22) Date of filing: **19.08.2015**

(86) International application number:
**PCT/JP2015/073266**

(87) International publication number:
**WO 2016/031650 (03.03.2016 Gazette 2016/09)**

(54) **METHOD FOR ASSESSING DEPRESSIVE STATE AND DEVICE FOR ASSESSING DEPRESSIVE STATE**

VERFAHREN ZUR BEURTEILUNG EINES DEPRESSIVEN ZUSTANDES UND VORRICHTUNG ZUR BEURTEILUNG EINES DEPRESSIVEN ZUSTANDES

PROCÉDÉ D'ÉVALUATION D'ÉTAT DÉPRESSIF ET DISPOSITIF D'ÉVALUATION D'ÉTAT DÉPRESSIF

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.08.2014 JP 2014172075**

(43) Date of publication of application:
**05.07.2017 Bulletin 2017/27**

(73) Proprietors:
• **Toyobo Co., Ltd.**
**Osaka-shi**
**Osaka 530-8230 (JP)**
• **UNION TOOL CO.**
**Tokyo 140-0013 (JP)**

(72) Inventors:
• **ISHIMARU, Sonoko**
**Otsu-shi**
**Shiga 520-0292 (JP)**
• **KOMATSU, Yoko**
**Otsu-shi**
**Shiga 520-0292 (JP)**
• **SHINOZAKI, Ryo**
**Tokyo 140-0013 (JP)**

(74) Representative: **Vossius & Partner**
**Patentanwälte Rechtsanwälte mbB**
**Siebertstrasse 3**
**81675 München (DE)**

(56) References cited:
**WO-A2-2006/056907    JP-A- 2008 067 892
JP-A- 2008 520 311    JP-A- 2009 524 450
JP-A- 2013 233 256    US-A1- 2014 058 279**

• **CHENDI WANG ET AL: "An emotional analysis method based on heart rate variability", BIOMEDICAL AND HEALTH INFORMATICS (BHI), 2012 IEEE-EMBS INTERNATIONAL CONFERENCE ON, IEEE, 5 January 2012 (2012-01-05), pages 104-107, XP032449014, DOI: 10.1109/BHI.2012.6211518 ISBN: 978-1-4577-2176-2**
• **TOSHIKAZU SHINBA ET AL.: 'Stress and autonomic activity' SEISHIN IGAKU vol. 49, no. 11, 15 November 2007, pages 1173 - 1181, XP009500633**

EP 3 187 116 B1

## EP 3 187 116 B1

**Description**

TECHNICAL FIELD

The present invention relates to a depression state determination device, and particularly to a depression state determination device, using a pulsation interval of a subject, and an acceleration or an angular velocity associated with a movement of the subject.

BACKGROUND ART

According to survey of Ministry of Health, Labor and Welfare, the number of patients who visit medical institutions because of mental disorders has tended to largely increase, and above all, a mental disorder having a most morbidity in a survey result in 2011 is a depression. The depression denotes a case where a depressive state (hereinafter, referred to as a "depression state" is at a certain level or higher, or at a serious level, and the depressive state is a state where a depressive feeling such as feeling down, feeling miserable and the like is strong (Non-Patent Literature 1). Continuation of such a depression state for a long time has bad mental and physical influences. Therefore, it is important to find the depression state in an early stage, and to start treatment early.

Conventionally, for diagnosis of the depression state, a method has been employed, in which a psychiatrist performs medical inquiry, and, if for example, there are a predetermined number of items that fall under a diagnosis reference such as ICD-10 and DSM-IV, the relevant person is determined to be in the depression state. In recent years, as an auxiliary diagnosis method, a method has been put into practical use, in which an optical topography device that measures and images a cerebral cortex portion, using a weak near infrared ray is used to measure change in hemoglobin concentration in blood of the cerebral cortex of a subject when medical inquiry is being answered (Non-Patent Literature 2).

Moreover, in Patent Literature 1, there has been described a biological analysis device in which as biological information of a subject, heartbeat is acquired from a heartbeat data acquisition device, and an acceleration is acquired, based on an acceleration acquired from an acceleration data acquisition device. Each piece of the biological information acquired in time series is segmented by time, using a statistic method such as a t-test and the like, and the time series data of the biological information is evaluated in accordance with the segmented time.

Patent Literature 2 relates to an evaluator including a heart rate variability analyzer having a heart rate measuring instrument, a heartbeat interval measuring instrument, a frequency analyzer, a sympathetic/parasympathetic nervous system index analyzer, and a psychiatric symptom/psychotic disorder onset risk calculator, and a random number generation analyzer having a random number input unit, a random number score calculation unit, and a display unit. Frequency analysis of a heart rate measurement and a heartbeat interval measurement of a subject is performed to determine a high-frequency index, a low-frequency index and a ratio therebetween.

Patent Literature 3 relates to a depression detection system and to a method of detecting depressions. In order to provide an objective measure for a depression as well as for a relapse into depression, a depression detection system is suggested, the system comprising a patient-based device using an actimetric sensor unit adapted to measure a patient's activity and further comprising a signal processing unit adapted to obtain a depression detection result based on the measured activity of the patient.

CITATION LIST

PATENT LITERATURE

PATENT LITERATURE 1
Japanese Unexamined Patent Application Publication No. 2008-67892
PATENT LITERATURE 2
US 2014/058279 A1
PATENT LITERATURE 3
WO 2006/056907 A2

NON PATENT LITERATURE

NON PATENT LITERATURE 1

http://www.mhlw.go.jp/kokoro/speciality/data.html
NON PATENT LITERATURE 2
http://www.hitachi-medical.co.jp/products/nirs/index.html
NON PATENT LITERATURE 3
http://www.terumo-taion.jp/health/depression/02.html

SUMMARY OF INVENTION

TECHNICAL PROBLEM

However, while the invention described in Patent Literature 1 is a method for properly evaluating the biological information, using the statistic method, and is not an analysis method specialized for the determination of the depression state. Moreover, as for the test method using the optical topography device described in Non-Patent Literature 2, a medical institution capable of the test is limited, so that the method is not the test method that everyone can easily undergo.

Consequently, an object of the present invention is to provide a depression state determination device that can determine a depression state with ease and with a high accuracy.

SOLUTION TO PROBLEM

The present inventors have considered a method for enabling a depression state to be determined, using easily measurable biological information, and first, have paid attention to symptoms of a patient in the depression state. It is known that in the patient in the depression state, operation of autonomic nerve, which is made up of a sympathetic nervous system exhibiting a stimulated/excited state, and a parasympathetic nervous system exhibiting a relax state, is disturbed, as compared with healthy people. These pieces of data can be easily obtained by measuring an interval of heartbeats or pulses. Next, the present inventors have paid attention to a relationship between an autonomic nerve activity and an activity (an acceleration or an angular velocity) indicating a movement of a body of a subject, and have observed a heartbeat interval and the activity of the patient in the depression state and the healthy people in an awaking time zone. As a result, it has been found even in the activity at almost the same level, the patient in the depression state tends to have a shorter heartbeat interval than the healthy people. This means that while the healthy people are in a relax state when the activity is less, so that the parasympathetic nerve activity becomes dominant, in the patient in the depression state, the sympathetic nerve activity becomes dominant regardless of the less activity. From these consideration results, the present inventors have considered that the use of the fact that the activity state and the activity of the autonomic nerve are in a close relationship enables the depression state to be determined. As a result of performing consideration such as Verification 1 and the like described later, it has been found that values of factors resulting from multiplying/dividing an index indicating the state of the autonomic nerve activity such as a pulsation interval and the like by the activity tend to be different between the healthy people and the patient in the depression state, and the use of these factors has been perceived for determining the depression state.

The present invention relates to a depression state determination device comprising: a measuring part measuring a pulsation interval of a subject, and an acceleration or an angular velocity associated with a movement of the subject (that is hereinafter referred to as an "activity"); a processing part calculating a value obtained by a procedure including a step of converting the pulsation interval to a frequency spectrum (that is hereinafter referred to as a "power spectrum") and calculating a value of definite integral of a power spectrum from frequency Lf1 to Lf2 (that is hereinafter referred to as an "LF") and a value of definite integral of a power spectrum from frequency Hf1 to Hf2 (that is hereinafter referred to as an "HF"); and a determining part determining the subject to be a depression state when at least one of the following conditions [A] and [B] is satisfied:

[A] In an awaking time zone of the subject, at least one of the following formulas (1) to (3) is calculated and satisfied:

$$\text{the pulsation interval} \times \text{the activity} < C1 \quad \cdots \quad (1)$$

$$\text{HF} \times \text{the activity} < C2 \quad \cdots \quad (2)$$

$$(\text{LF/HF}) / \text{the activity} > C3 \quad \cdots \quad (3);$$

[B] In a sleeping time zone of the subject, at least one of the following formulas (4) to (6) is calculated and satisfied:

$$\text{the pulsation interval} \, / \, \text{the activity} < C4 \quad \cdots \quad (4)$$

$$\text{HF} \, / \, \text{the activity} < C5 \quad \cdots \quad (5)$$

$$(\text{LF/HF}) \times \text{the activity} > C6 \quad \cdots \quad (6);$$

wherein
the Hf1 > the Lf1, the Hf2 > the Lf2, and the C1 to C6 are constant number. By measuring the pulsation interval and the activity of the subject, and using the conditions of the formulas (1) to (6), whether or not the subject is in the depression state can be determined with ease and with a high accuracy. This enables early detection and early treatment of the depression state to be performed.

The depression state determination device of the present invention is adapted to perform the following method for determining a depression state , said method comprising the steps of: measuring a pulsation interval of a subject, and an acceleration or an angular velocity associated with a movement of the subject (that is hereinafter referred to as an "activity"); and determining the subject to be a depression state when at least one of the following conditions [A] and [B] is satisfied:

[A] In an awaking time zone of the subject, at least one of the following formulas (1) to (3) is calculated and satisfied:

$$\text{the pulsation interval} \times \text{the activity} < C1 \quad \cdots \quad (1)$$

$$\text{HF} \times \text{the activity} < C2 \quad \cdots \quad (2)$$

$$(\text{LF/HF}) \, / \, \text{the activity} > C3 \quad \cdots \quad (3);$$

[B] In a sleeping time zone of the subject, at least one of the following formulas (4) to (6) is calculated and satisfied:

$$\text{the pulsation interval} \, / \, \text{the activity} < C4 \quad \cdots \quad (4)$$

$$\text{HF} \, / \, \text{the activity} < C5 \quad \cdots \quad (5)$$

$$(\text{LF/HF}) \times \text{the activity} > C6 \quad \cdots \quad (6);$$

wherein
the LF is a value of definite integral of a power spectrum from frequency Lf1 to Lf2, the power spectrum is obtained by a procedure including a step of converting the pulsation interval to a frequency spectrum, the HF is a value of definite integral of the power spectrum from frequency Hf1 to Hf2, the Hf1 > the Lf1, the Hf2 > the Lf2, and the C1 to C6 are constant number. By measuring the pulsation interval and the activity of the subject, and using the conditions of the formulas (1) to (6), whether or not the subject is in the depression state can be determined with ease and with a high accuracy. This enables early detection and early treatment of the depression state to be performed.

In the depression state determination device of the present invention, the LF may be a value of definite integral of a power spectrum from frequency Lf1 to Lf2, wherein the power spectrum is obtained by converting the pulsation interval to a frequency spectrum and squaring the frequency spectrum, and the HF may be a value of definite integral of a power spectrum from frequency Hf1 to Hf2, wherein the power spectrum is obtained by converting the pulsation interval to a frequency spectrum and squaring the frequency spectrum.

In the depression state determination device of the present invention, it is preferred that the subject is determined to be a depression state when at least one of the formulas is satisfied in the condition [A] and at least one of the formulas is satisfied in the condition [B]. Using measurement data in both the awaking time zone and the sleeping time zone can increase a determination accuracy of the depression state.

In the depression state determination device of the present invention, it is preferred that the subject is determined to be a depression state when the formula (3) and at least one of the formulas (1) and (2) are satisfied in the condition [A] and the formula (6) and at least one of the formulas (4) and (5) are satisfied in the condition [B]. The formula (3) using a factor of a sympathetic nerve activity in the awaking time zone, and the formula (1) or (2) using a factor of a parasympathetic nerve activity in the awaking time zone, and further the formula (6) using the factor of the sympathetic nerve activity in the sleeping time zone, and the formula (4) or (5) using the factor of the parasympathetic nerve activity in the sleeping time zone are combined to perform the determination, which can further increase the determination accuracy of the depression state.

In the depression state determination device of the present invention, it is preferred that the subject is determined to be a depression state when at least one of the formulas is satisfied in the condition [B]. As compared with the determination method of the formulas (1) to (3) in the awaking time zone, the determination method of the formulas (4) to (6) in the sleeping time zone tends to exhibit a larger difference between the healthy people and the patient in the depression state, which makes the determination of the depression state easier.

In the depression state determination device of the present invention, it is preferred that the pulsation interval is an R-R interval between R waves in an electrocardiographic signal. Since in the RR interval, a peak of the signal clearly appears, the accuracy of the pulsation interval increases, so that misrecognition of a peak position hardly occurs.

It is preferred that the awaking time zone and the sleeping time zone is distinguished from each other by measuring an acceleration associated with a posture of the subject and comparing the acceleration with a predetermined value. According to this distinction method, since the self-report of the subject is not required, a test burden of the subject can be reduced. Moreover, an erroneous distinction of the awaking time zone and the sleeping time zone due to omission of the report or an erroneous report by the subject is restrained from occurring.

It is preferred that the acceleration used for distinguishing the awaking time zone and the sleeping time zone is an acceleration in a height direction associated with a posture of the subject. Since a value of the acceleration in the height direction differs between in the awaking time zone and in the sleeping time zone, this method is suitable for the distinction between the awaking time zone and the sleeping time zone.

It is preferred that the sleeping time zone is determined as a time zone which satisfies the following formula (9) and the awaking time zone is determined as a time zone which does not satisfy the following formula (9):

$$\text{a negative acceleration } T \geq C7 \quad \cdots \quad (9),$$

wherein

in the case where the acceleration in the height direction measured in a standing position of the subject is a positive value, the negative acceleration T is calculated by multiplying the acceleration in the height direction by -1, in the case where the acceleration in the height direction measured in the standing position of the subject is a negative value, the negative acceleration T is the negative value, and the C7 is a constant number. In this manner, magnitudes of the negative acceleration T and the predetermined value C7 are compared, using the formula (9), which enables the awaking time zone and the sleeping time zone to be easily distinguished.

It is preferred that the sleeping time zone is determined as the longest time zone among a time zone which satisfies the following formula (9) and the awaking time zone is determined as a time zone other than the sleeping time zone:

$$\text{a negative acceleration } T \geq C7 \quad \cdots \quad (9),$$

wherein

in the case where the acceleration in the height direction measured in a standing position of the subject is a positive value, the negative acceleration T is calculated by multiplying the acceleration in the height direction by -1, in the case where the acceleration in the height direction measured in the standing position of the subject is a negative value, the negative acceleration T is the negative value, and the C7 is a constant number. This distinction method is effective to the subject who can continuously sleep without waking up in a half-way time during sleeping.

It is preferred that the sleeping time zone is determined as a time zone which consecutively satisfies the following formula (9) for not less than a predetermined time and the awaking time zone is determined as a time zone other than the sleeping time zone:

$$\text{a negative acceleration } T \geq C7 \quad \cdots \quad (9),$$

wherein
in the case where the acceleration in the height direction measured in a standing position of the subject is a positive value, the negative acceleration T is calculated by multiplying the acceleration in the height direction by -1, in the case where the acceleration in the height direction measured in the standing position of the subject is a negative value, the negative acceleration T is the negative value, and the C7 is a constant number. Since in this distinction method, the sleeping time zones determined in a hashed manner are added up to thereby estimate the substantial sleeping time zone, this method is effective to the subject having insomnia of nocturnal awaking, who wakes up at half-way time during sleeping.

It is preferred that the acceleration used for distinguishing the awaking time zone and the sleeping time zone is a value after performing a morphology operation to an acceleration-time waveform. Performing the morphology operation allows a whole outline of the acceleration-time waveform to be extracted, which makes easier the distinction between the awaking time zone and the sleeping time zone.

It is preferred that the morphology operation is at least one of an opening processing and a closing processing performed in a predetermined duration. Since combining an expansion operation and a contraction operation in this manner makes it easier to extract the whole outline of the acceleration-time waveform, the awaking time zone and the sleeping time zone can be distinguished more easily.

It is preferred that the measuring part of the depression state determination device of the present invention is provided with an input means that inputs an awaking state or a sleeping state. This allows the awaking information and sleeping information to be input from the input means.

It is preferred that the measuring part of the depression state determination device of the present invention is provided with a body temperature measuring means. The patient in the depression state exhibits a tendency different from the healthy people in change of a body temperature as well. Thus, when body temperature data is acquired by the body temperature measuring means, and both of this body temperature data and the foregoing formulas (1) to (6) are used, the determination accuracy of the depression state determination device can be further increased.

It is preferred that the pulsation interval is an R-R interval between R waves in an electrocardiographic signal. Since in the R-R interval, a peak of the signal clearly appears, the accuracy of the pulsation interval increases, so that misrecognition of a peak position hardly occurs.

ADVANTAGEOUS EFFECTS OF INVENTION

According to a depression state determination device, whether or not a subject is in a depression state can be determined with ease and with a high accuracy. Thus, the device contributes to early detection and early treatment of the depression state.

BRIEF DESCRIPTION OF THE DRAWINGS

Fig. 1 shows an explanatory diagram of a power spectrum integral according to the present invention.
Fig. 2 shows a block diagram showing a configuration of a depression state determination device according to a first embodiment of the present invention.
Fig. 3 shows a block diagram showing a configuration of a depression state determination device according to a second embodiment of the present invention.
Fig. 4 shows a block diagram showing a configuration of a depression state determination device according to a third embodiment of the present invention.
Fig. 5 shows a block diagram showing a configuration of a depression state determination device according to a fourth embodiment of the present invention.
Fig. 6 shows a block diagram showing a configuration of a depression state determination device according to a fifth embodiment of the present invention.
Fig. 7 shows a block diagram showing a configuration of a depression state determination device according to a sixth embodiment of the present invention.

Fig. 8 shows diagram showing examples in which the formula (1) of the method for determining the depression state performed by the depression state determination device according to an embodiment of the present invention are applied to a subject.

Fig. 9 shows diagram showing examples in which the formula (2) of the method for determining the depression state performed by the depression state determination device of the present invention are applied to a subject.

Fig. 10 shows diagram showing examples in which the formula (3) of the method for determining the depression state performed by the depression state determination device of the present invention are applied to a subject.

Fig. 11 shows diagram showing examples in which the formula (4) of the method for determining the depression state performed by the depression state determination device of the present invention are applied to a subject.

Fig. 12 shows diagram showing examples in which the formula (5) of the method for determining the depression state performed by the depression state determination device of the present invention are applied to a subject.

Fig. 13 shows diagram showing examples in which the formula (6) of the method for determining the depression state performed by the depression state determination device of the present invention are applied to a subject.

Fig. 14 shows a diagram showing an acceleration measured by a method for determining a depression state performed by the depression state determination device of the present invention.

Fig. 15 shows a diagram showing an acceleration measured by a method for determining a depression state performed by the depression state determination device of the present invention.

DESCRIPTION OF THE PREFERRED EMBODIMENTS

A method for determining a depression state performed by the depression state determination device of the present invention comprises the steps of: measuring a pulsation interval of a subject, and an activity associated with a movement of the subject; and determining the subject to be a depression state when at least one of the following conditions [A] and [B] is satisfied:

[A] In an awaking time zone of the subject, at least one of the following formulas (1) to (3) is calculated and satisfied:

$$\text{the pulsation interval} \times \text{the activity} < C1 \quad \cdots \quad (1)$$

$$\text{HF} \times \text{the activity} < C2 \quad \cdots \quad (2)$$

$$(\text{LF/HF}) / \text{the activity} > C3 \quad \cdots \quad (3);$$

[B] In a sleeping time zone of the subject, at least one of the following formulas (4) to (6) is calculated and satisfied:

$$\text{the pulsation interval} / \text{the activity} < C4 \quad \cdots \quad (4)$$

$$\text{HF} / \text{the activity} < C5 \quad \cdots \quad (5)$$

$$(\text{LF/HF}) \times \text{the activity} > C6 \quad \cdots \quad (6);$$

wherein
the LF is a value of definite integral of a power spectrum from frequency Lf1 to Lf2, the power spectrum is obtained by a procedure including a step of converting the pulsation interval to a frequency spectrum, the HF is a value of definite integral of the power spectrum from frequency Hf1 to Hf2, the Hf1 > the Lf1, the Hf2 > the Lf2, and the C1 to C6 are constant number. By measuring the pulsation interval and the activity of the subject, and using the conditions of the formulas (1) to (6), whether or not the subject is in the depression state can be determined with ease and with a high accuracy. This enables early detection and early treatment of the depression state to be performed.

Further, a depression state determination device of the present invention comprises: a measuring part measuring a pulsation interval of a subject, and an activity associated with a movement of the subject; a processing part calculating a value obtained by a procedure including a step of converting the pulsation interval to a frequency spectrum (that is hereinafter referred to as a "power spectrum") and calculating a value of definite integral of a power spectrum from frequency Lf1 to Lf2 (that is hereinafter referred to as an "LF") and a value of definite integral of a power spectrum from frequency Hf1 to Hf2 (that is hereinafter referred to as an "HF"); and a determining part determining the subject to be a depression state when at least one of the following conditions [A] and [B] is satisfied:

[A] In an awaking time zone of the subject, at least one of the following formulas (1) to (3) is calculated and satisfied:

$$\text{the pulsation interval} \times \text{the activity} < C1 \quad \cdots \quad (1)$$

$$HF \times \text{the activity} < C2 \quad \cdots \quad (2)$$

$$(LF/HF) / \text{the activity} > C3 \quad \cdots \quad (3);$$

[B] In a sleeping time zone of the subject, at least one of the following formulas (4) to (6) is calculated and satisfied:

$$\text{the pulsation interval} / \text{the activity} < C4 \quad \cdots \quad (4)$$

$$HF / \text{the activity} < C5 \quad \cdots \quad (5)$$

$$(LF/HF) \times \text{the activity} > C6 \quad \cdots \quad (6);$$

wherein
the Hf1 > the Lf1, the Hf2 > the Lf2, and the C1 to C6 are constant number. By measuring the pulsation interval and the activity of the subject, and using the conditions of the formulas (1) to (6), whether or not the subject is in the depression state can be determined with ease and with a high accuracy. This enables early detection and early treatment of the depression state to be performed.

1. Method for determining a depression state

In the method for determining a depression state performed by the depression state determination device of the present invention, a pulsation interval of a subject, and an activity which is an acceleration or an angular velocity associated with a movement of the subject are measured.
The pulsation interval denotes an interval of heartbeat or pulse (unit: ms). The heartbeat interval is acquired by reading an interval between R waves from an electrocardiogram, or measuring an interval between adjacent heartbeats. The pulse interval is acquired by measuring an interval between the adjacent pulses. The pulsation interval or swing thereof is said to indicate an autonomic nerve activity.
It is preferred that the pulsation interval is an R-R interval (that is hereinafter referred to as an "RRI") between R waves in an electrocardiographic signal. Since in the R-R interval, a peak of the signal clearly appears, the accuracy of the pulsation interval increases, so that misrecognition of a peak position hardly occurs.
The activity is the acceleration or the angular velocity associated with the movement of the subject, and is represented by a ratio to a gravitational acceleration g (unit: dimensionless quantity). Here, the acceleration is a value obtained by subtracting the gravitational acceleration g (= 9. 8 m/s$^2$) from a square root of a sum of squares of accelerations x, y, z in an X axis direction, in a Y axis direction, and in a Z axis direction (here, the unit g represents a magnitude of the gravitational acceleration). Accordingly, the acceleration A is represented by the following formula (7), so that the acceleration when there is no movement of the subject is zero, and the acceleration when the subject does some activity is larger than 0.

$$A = \sqrt{x^2 + y^2 + z^2} - 1 \quad \cdots \quad (7)$$

On the other hand, an angular velocity $\Omega$ is a square root of a sum of squares of angular velocities $\omega_X$, $\omega_y$, $\omega_z$ around the X axis, the Y axis, and the Z axis of the subject, and a unit is rad/s or 1/s. That is, the angular velocity $\Omega$ is represented by the following formula (8).

$$\Omega = \sqrt{\omega_x^2 + \omega_y^2 + \omega_z^2} \quad \cdots \quad (8)$$

Since the angular velocity detects rotation, for example, it is suitable for detecting a frequency of roll-over or the like of the subject in the sleeping time zone.

The method for determining the depression state performed by the depression state determination device of the present invention is roughly divided into a determination method in the awaking time zone (the condition [A]), and a determining method in the sleeping time zone (the condition [B]). The awaking time zone denotes a time zone when the subject is awake, that is, when the subject stays awake. On the other hand, the sleeping time zone denotes a time zone when the subject is sleeping, and time zones other than the awaking time zone. The awaking time zone and the sleeping time zone indicate various aspects from life environments and mental states of the subject, so that a time length and a time zone thereof are not particularly limited.

In the present invention, the distinction between the awaking time zone and the sleeping time zone may be performed by self-report of a sleeping start time and an awaking start time through a questionnaire to the subject, or may be performed by an input means provided in a depression state determination device described later. In addition, a publicly known method for measuring a sleeping state described, for example, in JP-A-2010-179133 and JP-A-2009-297474, or the like can also be applied.

It is preferred that the awaking time zone and the sleeping time zone is distinguished from each other by measuring an acceleration associated with a posture of the subject and comparing the acceleration with a predetermined value. According to this distinction method, since the self-report of the subject is not required, a test burden of the subject can be reduced. Moreover, an erroneous distinction of the awaking time zone and the sleeping time zone due to omission of the report or an erroneous report by the subject is restrained from occurring.

Generally, in the case where an accelerometer is adjusted so that the acceleration in a height direction in the sleeping time zone takes a negative value at the standing time, the acceleration in the height direction in the sleeping time zone tends to be larger than the acceleration in the height direction in the awaking time zone. Thus, it is preferred that the acceleration used for distinguishing the awaking time zone and the sleeping time zone is an acceleration in a height direction associated with a posture of the subject. Since a value of the acceleration in the height direction differs between in the awaking time zone and in the sleeping time zone, this method is suitable for the distinction between the awaking time zone and the sleeping time zone. The height direction in the present invention is a direction from a foot part of the subject toward a head part thereof.

The distinction between the awaking time zone and the sleeping time zone using the acceleration in the height direction can be specifically performed as follows. A first distinction method is a method in which a time zone when a formula (9) is satisfied is determined as the sleeping time zone, and a time zone when the formula (9) is not satisfied is determined as the awaking time zone. In the distinction method of the awaking time zone and the sleeping time zone described below, in the case where the acceleration in the height direction measured in a standing position of the subject is a positive value, the negative acceleration T is calculated by multiplying the acceleration in the height direction by -1, in the case where the acceleration in the height direction measured in the standing position of the subject is a negative value, the negative acceleration T is the negative value, and the C7 is a constant number.

$$T \geq C7 \quad \cdots \quad (9)$$

In this manner, magnitudes of the negative acceleration T and the predetermined value C7 are compared, using the formula (9), which enables the awaking time zone and the sleeping time zone to be easily distinguished. This method is suitable for a case where the awaking time zone and the sleeping time zone need to be distinguished in real time.

A second distinction method of the awaking time zone and the sleeping time zone using the negative acceleration T is a method in which the sleeping time zone is determined as the longest time zone among a time zone which satisfies the following formula (9) and the awaking time zone is determined as a time zone other than the sleeping time zone. In particular, the sleeping time zone is determined as the longest time zone among a time zone which satisfies the following formula (9) in a measurement unit time and the awaking time zone is determined as a time zone other than the sleeping time zone. The measurement unit time is a time length from measurement start to measurement end. In the present method, since the longest time zone when the formula (9) is satisfied is determined as the sleeping time zone, at this time, the measurement unit time is a time length within 24 hours inclusive so that at least one sleeping time zone is obtained in a day. The second distinction method is effective to the subject who can continuously sleep without waking up in a half-way time during sleeping.

A third distinction method of the awaking time zone and the sleeping time zone using the negative acceleration T is a method in which the sleeping time zone is determined as a time zone which consecutively satisfies the following formula (9) for not less than a predetermined time and the awaking time zone is determined as a time zone other than the sleeping time zone. The predetermined time is a time length set to consider, as the awaking time zone, a time zone when it is estimated that the subject is in a recumbent position in a state other than sleeping time. The predetermined time can be set to, for example, 15 minutes or longer, preferably, 30 minutes or longer, and more preferably, one hour or longer. Since in the third distinction method, the sleeping time zones determined in a hashed manner are added up to thereby estimate the substantial sleeping time zone, this method is effective to the subject having insomnia of nocturnal awaking, who wakes up at half-way time during sleeping.

In the case of the distinction the awaking time zone and the sleeping time zone by using the acceleration, it is preferred that the acceleration is a value after performing a morphology operation to an acceleration-time waveform. The morphology operation is used for noise removal in an image processing. Therefore, if the value after the morphology operation is performed to the acceleration-time waveform is applied to the formula (9), a value that changes in a short time (e.g., within 1/150 hours of a total measurement time inclusive) as compared with the total measurement time, is removed from the obtained acceleration. Thus, a whole outline of the acceleration-time waveform is extracted so that it is easier to distinguish between the awaking time zone and the sleeping time zone. As a result, since the number of times of determination by the formulas (1) to (6) can be decreased, the time required for determination of the depression state can be shortened.

In order to shorten a processing time required for the morphology operation, it is also preferred that the morphology operation is performed to the acceleration-time waveform subjected to binarization processing with the predetermined value C7 used as a threshold value. In the binarization processing, for example, if the acceleration is the predetermined value (the threshold value) C7 or more, the acceleration is considered to be 0, while if the acceleration is less than the predetermined value C7, the acceleration is considered to be 1.

As the morphology operation, there are, for example, an expansion operation to perform a processing for thickening a line, a contraction operation to perform a processing for thinning a line, an opening processing of performing the expansion operation after the contraction operation, and a closing processing of performing the contraction operation after the expansion operation. In the present invention, in the case where the awaking time zone and the sleeping time zone are distinguished, using the acceleration after the morphology operation, it is preferred that the morphology operation is at least one of the opening processing and the closing processing performed in a predetermined duration. Moreover, as the morphology operation, it is more preferred that both the opening processing and closing processing are performed. Since combining an expansion operation and a contraction operation in this manner makes it easier to extract the whole outline of the acceleration-time waveform, the awaking time zone and the sleeping time zone can be distinguished more easily.

While numbers of times of the opening processing and the closing processing are not particularly limited, preferably, each of the opening processing and closing processing is performed once or more, and more preferably, each of the opening processing and the closing processing is performed twice or more.

A time width when the expansion operation and the contraction operation are performed may be set as needed, and for example, the time width of the first opening processing and closing processing can be set to 2 minutes, and the time width of the second opening processing and closing processing can be set to 5 minutes. In this manner, it is preferred that every time the number of times of the processing is increased, the time width at the time of processing is increased. Thus, increasing the time width of the opening processing and the closing processing in stages restrains the data of the acceleration changing in a shorter time, as compared with the total measurement time from being removed.

The LF is a value of definite integral of a power spectrum from frequency Lf1 to Lf2, the power spectrum is obtained by a procedure including a step of converting the pulsation interval which is time signal f to a frequency spectrum, the HF is a value of definite integral of the power spectrum from frequency Hf1 to Hf2, the Hf1 > the Lf1, the Hf2 > the Lf2, and the C1 to C6 are constant number. For example, the LF may be a value of definite integral of a power spectrum $F^2$ (a first power spectrum) from frequency Lf1 to Lf2. The power spectrum is obtained by converting the pulsation interval which is time signal f to a frequency spectrum (a frequency spectrum F) and squaring the frequency spectrum, and the HF may be a value of definite integral of the power spectrum $F^2$ (the first power spectrum) from frequency Hf1 to Hf2. A unit of the LF, the HF, calculated, using the first power spectrum $F^2$ is $ms^2$. Specific values of C1 to C6 are not particularly limited, but can be set in accordance with an integral range of the power spectrum integral, a type of the activity (the acceleration or the angular velocity), conditions of the subject such as an age, a gender and the like. As a frequency spectrum conversion method, for example, fast Fourier transform (FFT), wavelet analysis, a maximum entropy method, and the like can be used. While in the present specification, a case using FFT is described as an example, obviously, another method can also be used.

In the present specification, discrete Fourier transform $G_k$ of a pulsation interval $RRI_k$ obtained by subjecting the pulsation interval to spline interpolation and resampling at a sampling interval $\Delta t$ is represented by the following formula (10), and the power spectrum $F^2$ (the first power spectrum) (unit: ms$^2$/Hz) is represented by the following formula (11). Here, k denotes time series, N denotes the number of pieces of data, and S denotes an arbitrary scale, and generally, S = 1 in the power spectrum.

$$G_k = \sum_{k=0}^{N-1} RRI_k \exp\left(2\pi i f k \Delta t\right) \quad \cdots \quad (10)$$

$$F^2 = S\frac{2\Delta t}{N}G^2 \quad \cdots \quad (11)$$

On the other hand, a method can be included in the method for determining the depression state performed by the depression state determination device of the present invention, in which as the values of the LF and the HF, a power spectrum F (a second power spectrum) (unit: ms) obtained from the value resulting from frequency spectrum conversion of the pulsation interval is subjected to definite integral in a predetermined period. In this manner, the use of the value obtained by subjecting the pulsation interval to the frequency spectrum conversion as the power spectrum enables the values of the LF and the HF to be more easily calculated. The unit of the LF, the HF, calculated, using the second power spectrum F is dimensionless quantity. The power spectrum F (the second power spectrum) is represented by the following formula (12).

$$F = S\frac{2}{N}|G| \quad \cdots (12)$$

Hereinafter, details of the LF, the HF, and C1 to C6 will be described.

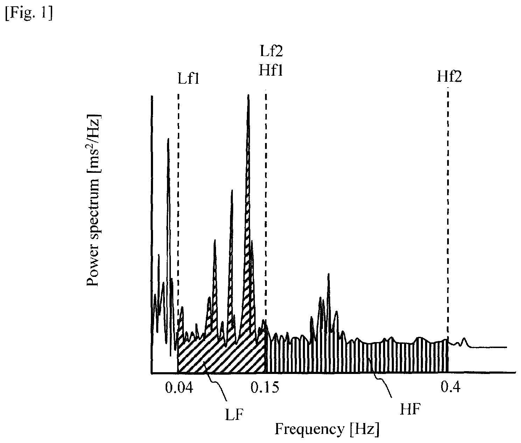

Fig. 1 shows an explanatory diagram of the power spectrum integral of the present invention. A vertical axis of Fig. 1 indicates the power spectrum (unit: ms$^2$/Hz), and a horizontal axis indicates a frequency (unit: Hz). The LF is a value of the definite integral of the power spectrum $F^2$, for example, from 0.04 Hz (Lf1) to 0.15 Hz (Lf2), and indicates an area of a portion hatched by oblique lines in Fig. 1. On the other hand, the HF is a value of the definite integral of the power spectrum $F^2$, for example, from 0.15 Hz (Hf1) to 0.4 Hz (Hf2), and indicates an area of a portion hatched by vertical lines in Fig. 1. While in Fig. 1, an integral range is set so that the Lf2 and the Hf1 are equal, that is, 0.15 Hz, the Lf2 and the Hf1 may be different values, as long as a relationship of Lf1 < Hf1 and Lf2 < Hf2 is satisfied. While here, the method of the power spectrum integral has been described, using the first power spectrum $F^2$, the definite integral by the second power spectrum F can be similarly performed.

The power spectrum obtained by the frequency spectrum conversion is classified into the LF, which is a component derived from fluctuation of a blood pressure, and is also referred to as a Mayer-Wave related component, and the component HF derived from respiration. The blood pressure fluctuation component LF is the power spectrum of around 0.1 Hz, and is related to both the sympathetic nervous system and the parasympathetic nervous system. On the other hand, the component HF derived from respiration is the power spectrum of around 0.3 Hz, and is considered to be related to the parasympathetic nervous system.

From the foregoing, it is preferred that the integral range of the LF indicating a sympathetic nerve activity and a parasympathetic nerve activity includes at least 0.1 Hz, and Lf1 < 0.1 < Lf2. More preferably, the Lf1 is 0.03 Hz, and still more preferably, 0.04 Hz. More preferably, the Lf2 is 0.16 Hz, and still more preferably 0.15 Hz.

Also, it is preferred that the integral range of the HF indicating a parasympathetic nerve activity includes at least 0.3 Hz, and Hf1 < 0.3 < Hf2. More preferably, the Hf1 is 0.14 Hz, and still more preferably 0.15 Hz. More preferably, the Hf2 is 0.41 Hz, and still more preferably 0.4 Hz.

In the method for determining the depression state performed by the depression state determination device of the present invention, it is determined that the subject is in the depression state if the pulsation interval $\times$ the activity < C1 is satisfied in the awaking time zone of the subject. As described above, the pulsation interval indicates the parasympathetic nerve activity, and the activity indicates the movement of the subject. For example, when the activity is the acceleration, C1 is preferably 150 ms, more preferably, 160 ms, and still more preferably, 170 ms. As to the pulsation interval $\times$ the activity, a difference between the healthy people and the patient in the depression state becomes largest when C1 is 150 ms or more, and thus, the pulsation interval $\times$ the activity is a good index for determining the depression state. In order to make the determination of the depression state easier, the formula (1) may be the pulsation interval $\times$ the activity/100 < C11. At this time, C11 is a value obtained by dividing C1 by 100, that is, C11 = C1/100.

In the method for determining the depression state performed by the depression state determination device of the present invention, it is determined that the subject is in the depression state if the HF $\times$ the activity < C2 is satisfied in the awaking time zone of the subject. As described above, the HF indicates the parasympathetic nerve activity as well as the pulsation interval. The formula (2) is to determine the depression state from the parasympathetic nerve activity and the activity as with the formula (1). For example, if the acceleration is used as the activity, and the integral range of the HF obtained from the first power spectrum $F^2$ is set to 0.15 Hz to 0.4 Hz, C2 is preferably 0.08 $ms^2$, more preferably 0.085 $ms^2$, and still more preferably, 0.09 $ms^2$. In order to make the determination of the depression state easier, the formula (2) may be the HF $\times$ the activity $\times$ 100 < C21. At this time, C21 is represented by a value obtained by multiplying C2 by 100, that is, C21 = C2 $\times$ 100.

On the other hand, if the acceleration is used as the activity, and the integral range of the HF obtained from the second power spectrum F is set to 0.15 Hz to 0.4 Hz, C2 is preferably 80, more preferably, 90, and still more preferably 100. Here, a unit of C2 is dimensionless quantity.

In the method for determining the depression state performed by the depression state determination device of the present invention, it is determined that the subject is in the depression state if (the LF / the HF) / the activity > C3 is satisfied in the awaking time zone of the subject. As described above, since the LF indicates both the sympathetic nerve activity and the parasympathetic nerve activity, LF/HF expresses predominance of the sympathetic nerve activity to the parasympathetic never activity. Accordingly, LF/HF in the present invention is used as an index indicating the sympathetic nerve activity. The formula (3) is a method for determining the depression state from the sympathetic nerve activity and the activity. For example, if the acceleration is used as the activity, and the integral range of the LF obtained from the first power spectrum $F^2$ is set to 0.04 Hz to 0.15 Hz, C3 is preferably 5, more preferably 4.5, and still more preferably 4. Here, a unit of C3 is dimensionless quantity.

On the other hand, if the acceleration is used as the activity, and the integral range of the LF obtained from the second power spectrum F is set to 0.04 Hz to 0.15 Hz, and the integral range of the HF obtained from the second power spectrum F is set to 0.15 Hz to 0.4 Hz, C3 is preferably 27, more preferably 26, and still more preferably 25. Here, a unit of C3 is dimensionless quantity.

In the method for determining the depression state performed by the depression state determination device of the present invention, it is determined that the subject is in the depression state if the pulsation interval / the activity < C4 is satisfied in the sleeping time zone of the subject. The formula (4) is a method for determining the depression state from the parasympathetic never activity and the activity. For example, if the acceleration is used as the activity, C4 is preferably 1200ms, more preferably 1500ms, and still more preferably 1900ms. In order to make the determination of the depression state easier, the formula (4) may be (the pulsation interval / the activity) / 100 < C41. At this time, C41 is represented by a value obtained by dividing C4 by 100, that is, C41 = C4 / 100.

In the method for determining the depression state performed by the depression state determination device of the present invention, it is determined that the subject is in the depression state if the HF / the activity < C5 is satisfied in the sleeping time zone of the subject. The formula (5) is a method for determining the depression state from the parasympathetic never activity and the activity. For example, if the acceleration is used as the activity, and the integral range of the HF obtained from the first power spectrum $F^2$ is set to 0.15 Hz to 0.4 Hz, C5 is preferably 40$ms^2$, more preferably 50$ms^2$, still more preferably 60$ms^2$, and most preferably 70$ms^2$.

On the other hand, if the acceleration is used as the activity, and the integral range of the HF obtained from the second power spectrum F is set to 0.15 Hz to 0.4 Hz, C5 is preferably 30000, more preferably 40000, and still more preferably 50000. Here, a unit of C5 is dimensionless quantity. In order to make the determination of the depression state easier, the formula (5) may be (the HF / the activity) / 1000 < C51. At this time, C51 is represented by a value obtained by dividing C5 by 1000, that is, C51 = C5 / 1000.

In the method for determining the depression state performed by the depression state determination device of the present invention, it is determined that the subject is in the depression state if (the LF / the HF) $\times$ the activity > C6 is satisfied in the sleeping time zone of the subject. The formula (6) is a method for determining the depression state from the sympathetic nerve activity and the activity. For example, if the acceleration is used as the activity, the integral range of the LF obtained from the first power spectrum $F^2$ is set to 0.04 Hz to 0.15 Hz, and the integral range of the HF obtained from the first power spectrum $F^2$ is set to 0.15 Hz to 0.4 Hz, C6 is preferably 0.015, more preferably 0.01, and still more preferably 0.005. Here, a unit of C6 is dimensionless quantity. In order to make the determination of the depression state easier, the formula (6) may be (the LF / the HF) $\times$ the activity $\times$ 100 > C61. At this time, C61 is represented by a value obtained by multiplying C6 by 100, that is, C61 = C6 $\times$ 100.

On the other hand, if the acceleration is used as the activity, the integral range of the LF obtained from the second power spectrum F is set to 0.04 Hz to 0.15 Hz, and integral range of the HF obtained from the second power spectrum F is set to 0.15 Hz to 0.4 Hz, the C6 is preferably 0.045, more preferably 0.03, and still more preferably 0.015.

Here, a unit of C6 is dimensionless quantity. In order to make the determination of the depression state easier, the formula (6) may be (the LF / the HF) $\times$ the activity $\times$ 100 > C61. At this time, C61 is represented by a value obtained by multiplying C6 by 100, that is, C61 = C6 $\times$ 100.

In the method for determining the depression state performed by the depression state determination device of the present invention, it is preferred that the subject is determined to be a depression state when at least one of the formulas is satisfied in the condition [A] and at least one of the formulas is satisfied in the condition [B]. That is, it is preferred that the subject is determined to be in the depression state when at least one of the formulas (1) to (3) is satisfied and at least one of the formulas (4) to (6) is satisfied. Using measurement data in both the awaking time zone and the sleeping time zone can increase a determination accuracy of the depression state.

In the method for determining a depression state performed by the depression state determination device of the present invention, it is preferred that the subject is determined to be a depression state when the formula (3) and at least one of the formulas (1) and (2) are satisfied in the condition [A] and the formula (6) and at least one of the formulas (4) and (5) are satisfied in the condition [B]. The formula (3) using a factor of a sympathetic nerve activity in the awaking time zone, and the formula (1) or (2) using a factor of a parasympathetic nerve activity in the awaking time zone, and further the formula (6) using the factor of the sympathetic nerve activity in the sleeping time zone, and the formula (4) or (5) using the factor of the parasympathetic nerve activity in the sleeping time zone are combined to perform the determination, which can further increase the determination accuracy of the depression state.

In the method for determining a depression state performed by the depression state determination device of the present invention, it is preferred that the subject is determined to be a depression state when at least one of the formulas is satisfied in the condition [B]. As compared with the determination method of the formulas (1) to (3) in the awaking time zone, the determination method of the formulas (4) to (6) in the sleeping time zone tends to exhibit a larger difference between the healthy people and the patient in the depression state, which makes the determination of the depression state easier.

Combining the formulas (1) to (6) as needed enables the determination of the depression state to be performed. For example, if only the formulas (1) and (4), which use only the pulsation interval and the activity, are used for the determination of the depression state, the steps of performing the frequency spectrum conversion and the power spectrum integral can be omitted. Thus, it is effective to a case where a simpler determination method is desired. On the other hand, if all of the formulas (1) to (6) are used to perform the determination, the accuracy of the determination result can be increased.

As a result from performing the evaluation of all the formulas (1) to (6), if at least one of the formulas is satisfied, the subject can be determined to be in the depression state, and also, if all the formulas are satisfied, the subject can be determined to be in the depression state. Accordingly, the number of the formulas used for the determination is not particularly limited. In the method in which if at least one of the formulas (1) to (6) is satisfied, the subject is determined to be in the depression state, the patient who is a suspected case of the depression state in a wider range can be screened. Moreover, in the method in which if all the formulas (1) to (6) are satisfied, the subject is determined to be in the depression state, the determination of the depression state can be performed with a high accuracy.

2. Depression state determination device

The depression state determination device of the present invention includes a measuring part, a processing part, and a determining part. The measuring part is an electrocardiograph or a heartbeat sensor that measures the heartbeat of the subject, or a pulse wave sensor that measures a pulse wave of the subject to find the pulse, an acceleration sensor or an angular velocity sensor that measures the activity of the subject, and the like. The processing part performs the frequency spectrum conversion, based on the pulsation interval measured by the measuring part, and calculates the power spectrum integrated value to calculate the LF and the HF used for the determination of the depression state. The determining part prepares determination data, using the pulsation interval and the activity measured by the measuring part, and the LF and the HF obtained by the processing part, and compares the predetermined values C1 to C6 with the determination data to perform the determination of the depression state. The processing part and the determining part are a computer, measurement equipment or the like mounting software that performs the creation of the determination data used for the determination of the depression state, the comparison between the predetermined values and the determination data, and the like.

(First Embodiment)

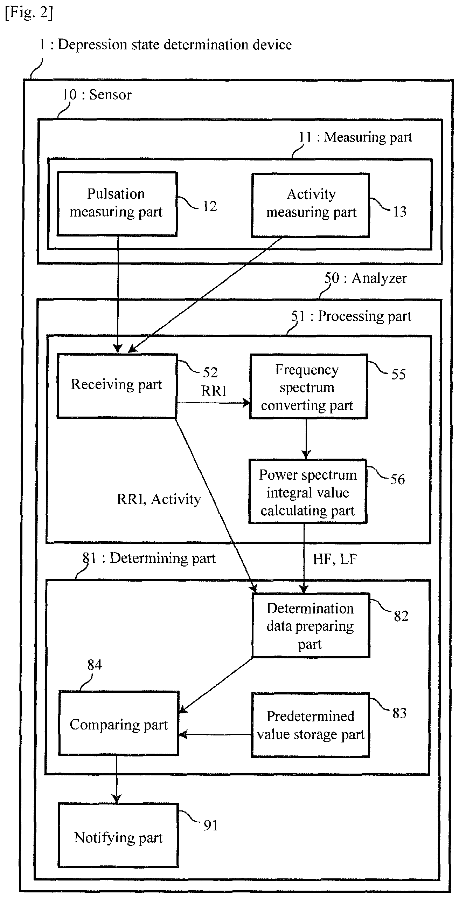

Fig. 2 is a block diagram showing a configuration of a depression state determination device 1 according to a first embodiment of the present invention. The depression state determination device 1 shown in Fig. 2 includes a sensor 10 as a measuring part and an analyzer 50.

(1) Measuring part

The sensor 10 includes a measuring part 11 configured by a pulsation measuring part 12 that detects the pulsation interval, and an activity measuring part 13 that detects the activity. The sensor 10 is small and lightweight, and since the whole body thereof can be attached to skin of the subject in a state where electrodes (not shown) in a body back surface are brought into close contact with a chest of the subject, the sensor 10 is not noticeable because it is concealed under clothes. It is preferred that the pulsation interval is an R-R interval between R waves in an electrocardiographic signal. Since in the R-R interval, a peak of the signal clearly appears, the accuracy of the pulsation interval increases, so that misrecognition of a peak position hardly occurs.

In the present embodiment, the RRI is measured as the pulsation interval, and the acceleration is measured as the activity.

The pulsation measuring part 12 measures an electrocardiographic signal in the state where the electrodes are brought into close contact with the chest of the subject, and calculates the RRI, based on this electrocardiographic signal to transmit the resultant to the analyzer 50. While the pulsation measuring part 12 of the sensor 10 calculates the RRI, based on the electrocardiographic signal, the calculation of the RRI may be performed in the processing part 51 described later.

In the pulsation measuring part 12, a pulse wave may be measured in place of the heartbeat. The pulse wave can be measured by irradiating a human fingertip, earlobe or the like with near infrared rays having a wavelength of 700 nm to 1200 nm and measuring the amount of reflection of the near infrared rays in a contact or contactless manner. Measuring the pulse wave has an advantage that a measuring instrument can be relatively easily attached to a body. Especially, since if the measuring instrument that measures the pulse wave in a non-contact state is used, troublesomeness of attaching the measuring instrument to the body is eliminated, there may be widely used. The pulsation interval can be found from an interval between adjacent peaks of the pulse wave measured in this manner.

In the activity measuring part 13 of the measuring part 11, accelerations in an X axis direction, in a Y axis direction, and in a Z axis direction of the subject are measured to be transmitted to the analyzer 50. A type of the sensor measuring the acceleration is not particularly limited, and for example, a piezoresistor type acceleration sensor, a piezoelectric type acceleration sensor, an electrostatic capacity type acceleration sensor, or the like can be used. The piezoresistor type acceleration sensor is small and easy to mass-produce because it uses a semiconductor. The piezoelectric type acceleration sensor easily detects the relatively high acceleration. The electrostatic capacity type acceleration sensor has high sensitivity, a wide detectable range of the acceleration, and low temperature dependency, as compared with the piezoresistor type acceleration sensor.

As the activity, the angular velocity may be detected in place of the acceleration. A type of the sensor that measures the angular velocity is not particularly limited, but, for example, a rotary type, a vibration type, a gas type, an optical fiber type, or a ring laser type angular velocity sensor can be used.

As a method for transmitting the data of the pulsation interval and the activity measured in the measuring part 11 to a receiving part 52 of the analyzer 50, wireless communication may be used, or wired communication may be used. Especially, when the data is transmitted/received through the wireless communication, it is preferred that in order to increase a life of a built-in battery, for example, three RRIs are transmitted collectively or the like to decrease frequency of the transmission/reception. Moreover, it is preferred that at this time, the acceleration as the activity is transmitted/received at the same timing as that of the RRIs.

In view of suppression of power consumption, it is also preferred that the sensor according to the present invention is automatically turned off after a predetermined time has elapsed since the sensor was turned on. The predetermined time may be set in terms of the number of pieces of data required for the determination of the depression state, and for example, it may be set to 24 hours, 48 hours or the like.

(2) Processing part

The analyzer 50 includes the processing part 51 and a determining part 81, and the processing part 51 includes the receiving part 52, a frequency spectrum converting part 55, and a power spectrum integral value calculating part 56. The receiving part 52 receives the RRI and the activity transmitted from the sensor 10.

The frequency spectrum converting part 55 converts the RRI as a time signal transmitted from the receiving part 52 to a frequency spectrum, using the frequency spectrum conversion method such as FFT and the like. Next, the power spectrum integral value calculating part 56 calculates the power spectrum from the spectrum obtained in the frequency spectrum converting part 55, and performs integral in a predetermined frequency range to thereby find the LF and the HF. Specifically, the following processing is performed. First, when the power spectrum is calculated from the frequency spectrum obtained in the frequency spectrum converting part 55, a distribution diagram with a vertical axis indicating a power spectrum density, and with a horizontal axis indicating a frequency can be obtained. Next, the power spectrum is integrated in the range of the Lf1 to the Lf2, and in the range of the Hf1 to the Hf2 to thereby find the LF and the HF, respectively, where Lf1 < Hf1, Lf2 < Hf2. The specific calculation method of the power spectrum has been described in "1. Method for determining a depression state", and as the power spectrum, for example, the first power spectrum $F^2$ may be used, or the second power spectrum F may be used.

(3) Determining part

The determining part 81 of the depression state determination device of the present invention includes a determination data preparing part 82, a predetermined value storage part 83, and a comparing part 84. First, the determination data preparing part 82 prepares the determination data required for the formulas (1) to (6) used for the determination of the depression state. The RRI and the activity are transmitted from the receiving part 52 of the processing part 51, and the HF and the LF are transmitted from the power spectrum integral value calculating part 56 of the processing part 51 to the determination data preparing part 82 of the determining part 81. These pieces of data are multiplied/divided to thereby prepare the determination data described in left sides of the formulas (1) to (6).

The predetermined values C1 to C6 described in right sides of the formulas (1) to (6) used for the determination of the depression state are stored in the predetermined value storage part 83. It is preferred that the predetermined value storage part 83 is provided with an input means so that the predetermined values C1 to C6 can be changed as needed.

In the comparing part 84, the data for determination prepared in the determination data preparing part 82 (i.e., the data of the left sides of the formulas (1) to (6)), and the predetermined values stored in the predetermined value storage part 83 (i.e., the data of the right sides of the formulas (1) to (6)) are substituted into the formulas (1) to (6), and magnitudes of the left sides and the right sides are compared to determine whether or not the formulas (1) to (6) are satisfied. If the formulas (1) to (6) are satisfied, it is determined that the subject is in the depression state, and if the formulas (1) to (6) are not satisfied, it is determined that the subject is not in the depression state.

It is preferred that the analyzer 50 is provided with a notifying part 91 that notifies the subject or the like of a determination result of the depression state from the determining part 81. A notification method is not particularly limited, and a voice, a still picture, a moving picture and the like can be employed. A notification content can be changed in accordance with a specialized knowledge level of a notification object such as a specialist including a doctor, a counsellor and the like, the subject, his or her family and the like. While here, the example has been described in which the notifying part 91 is provided in the depression state determination device 1, the determination result may be transmitted to equipment for notification different from the depression state determination device 1 to notify the subject or the like of the result. As the equipment for notification, for example, an external monitor, a portable telephone, a smartphone, a tablet terminal, a speaker, an earphone, and the like are cited.

(Second Embodiment)

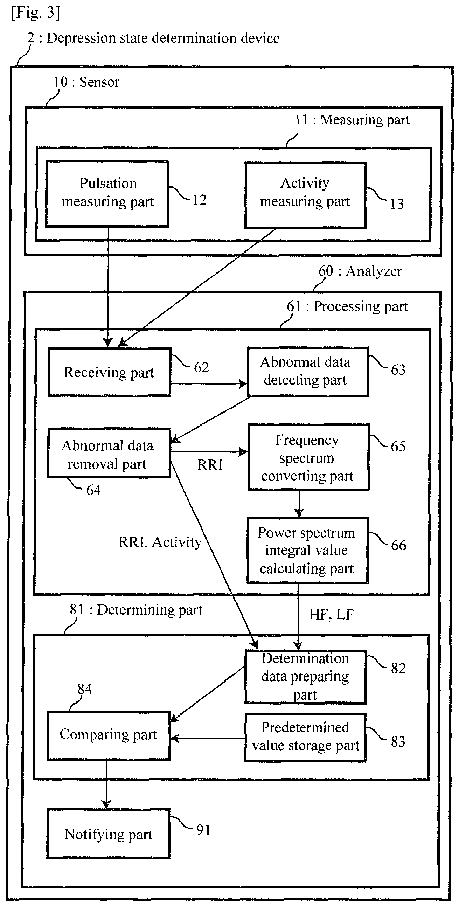

Fig. 3 is a block diagram showing a configuration of a depression state determination device 2 according to a second embodiment of the present invention. The depression state determination device 2 shown in Fig. 3 includes a sensor 10 and an analyzer 60. Note that the same components as those of the depression state determination device 1 of the first embodiment are denoted by the same reference signs and a description thereof is omitted.

The analyzer 60 includes the processing part 61 and a determining part 81, and the processing part 61 includes a receiving part 62, an abnormal data detecting part 63, an abnormal data removal part 64, a frequency spectrum converting part 65 and a power spectrum integral value 66.

The abnormal data detecting part 63 decides whether a RRI outputted from the receiving part 62 is to be considered to have an abnormal value. In the second embodiment, an instantaneous heart rate is calculated by multiplying an inverse of a RRI (units of seconds) by 60. An average of instantaneous heart rates at a plurality of recent points ("eight points" in the present embodiment) where an absolute value of the difference between an instantaneous heart rate and the previous instantaneous heart rate is not more than a first predetermined number ("18" in the present embodiment) is calculated. Then, when an absolute value of the difference between the average and an instantaneous heart rate corresponding to a RRI which is an evaluation object is not less than a second predetermined number ("35" in the present embodiment), the RRI which is the evaluation object is considered to have an abnormal value. Here, the first predetermined number is preferably 30, more preferably 20, and further more preferably 15. In addition, the second predetermined number is preferably 50, more preferably 40, and further more preferably 30.

Note that the first predetermined number, the second predetermined number, and the number of a plurality of recent points may be changed as appropriate, according to individual differences, etc. For example, the first predetermined number may be changed as appropriate to not more than 30, the second predetermined number to not less than 30, and the number of a plurality of recent points within a range of 4 to 20.

Since the activity becomes zero when there is no body movement, the value does not become a minus value. Accordingly, it is preferred that when the activity is a minus value, it is considered to be an abnormal value.

The abnormal data removal part 64 removes the RRI considered to be an abnormal values by the abnormal data detecting part 63 from an objects of the data processing in the frequency spectrum converting part 65. Moreover, the abnormal data removal part 64 removes the RRI and the activity considered to be abnormal values by the abnormal data detecting part 63 from objects of the data processing in the determination data preparing part 82.

(Third Embodiment)

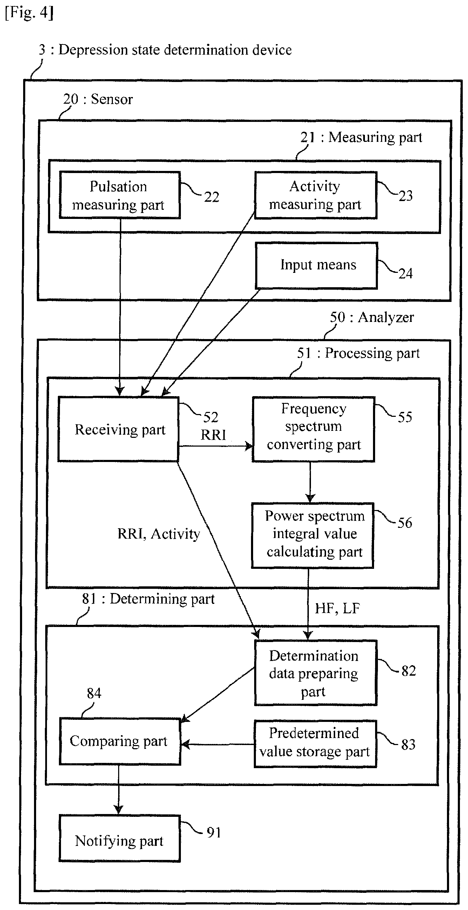

Fig. 4 is a block diagram showing a configuration of a depression state determination device 3 according to a third embodiment of the present invention. The depression state determination device 3 shown in Fig. 4 includes a sensor 20 and an analyzer 50. Note that the same components as those of the depression state determination device 1 of the first embodiment are denoted by the same reference signs and a description thereof is omitted.

A measuring part 21 of the sensor 20 is preferably provided with an input means 24 that inputs an awaking state or a sleeping state in addition to a pulsation measuring part 22 and an activity measuring part 23. Thereby, a subject operates the input means 24 by himself or herself at the start time of an awaking time zone and at the start time of a sleeping time zone, which can bring about awaking information and sleeping information. The input means 24 is, for example, a switch provided in a surface of the sensor, and this switch may be of a button type or of a lever type, so that a mode therefor is not particularly limited.

The input means 24 is also preferably provided in the analyzer 50 in place of the sensor 20 of the depression state determination device 3. This can prevent the subject from unconsciously operating the input means 24 with roll-over during sleeping or the like, as compared with the case where the input means 24 is provided in the sensor 20.

(Fourth Embodiment)

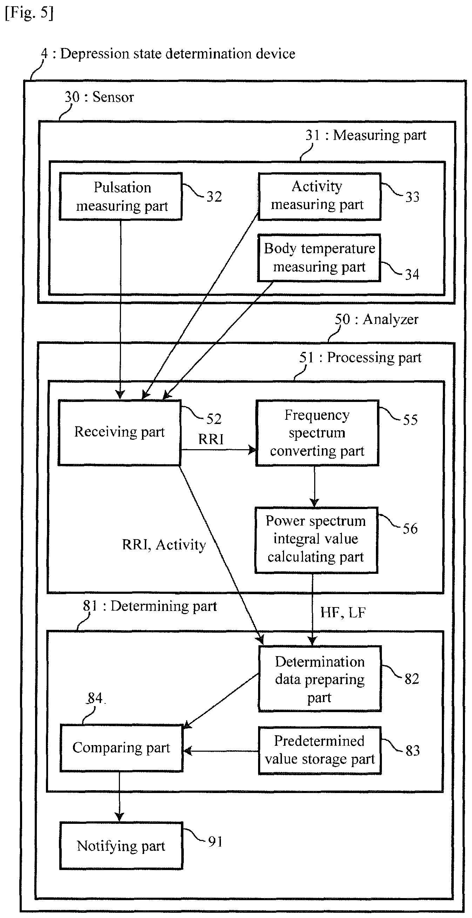

Fig. 5 is a block diagram showing a configuration of a depression state determination device 4 according to a fourth embodiment of the present invention. The depression state determination device 4 shown in Fig. 5 includes a sensor 30 and an analyzer 50. Note that the same components as those of the depression state determination device 1 of the first embodiment are denoted by the same reference signs and a description thereof is omitted.

A measuring part 31 of the sensor 30 is preferably provided with a body temperature measuring means as a body temperature measuring part 34 in addition to a pulsation measuring part 32 and an activity measuring part 33. It is known that in the case of the depression state, the patient is likely to suffer hypothermia. It is because that in the depression state, the patient is likely to have a nocturnal lifestyle, thereby reducing an opportunity of exposure to the sun, which makes it hard to keep a biological clock normal. Furthermore, since the patient in the depression state has a high temperature even at night, the patient is in a state where his or her body is not rested (Non-Patent Literature 3). Thus, when body temperature data is acquired by the body temperature measuring means (the body temperature measuring part 34), and both of this body temperature data and the foregoing formulas (1) to (6) are used, the determination accuracy of the depression state determination device can be further increased.

Generally, a body temperature is measured in a deep part of a body such as in an oral cavity, underarm, at an eardrum and the like, which has a small influence by an environmental temperature. However, since the measuring part of the depression state determination device of the present invention is attached to the skin of the subject to measure the heartbeat or the pulse wave, it is hard to directly measure the deep body temperature. Accordingly, the body temperature here includes not only the deep body temperature but a body surface temperature. Moreover, it is also possible to measure a temperature of a substrate of a member configuring the measuring part, for example, the sensor, in place of directly measuring the body temperature. Since if the body temperature of the subject rises, the substrate temperature of the sensor also rises, measuring the substrate temperature of the sensor allows change of the body temperature to be relatively measured. A type of body temperature measurement means is not particularly limited, as long as it is a temperature sensor, and for example, a metal temperature sensing resistor of platinum, nickel, copper or the like, a thermocouple, a thermistor, an integrated circuit temperature sensor, a quartz thermometer, or the like can be used.

(Fifth Embodiment)

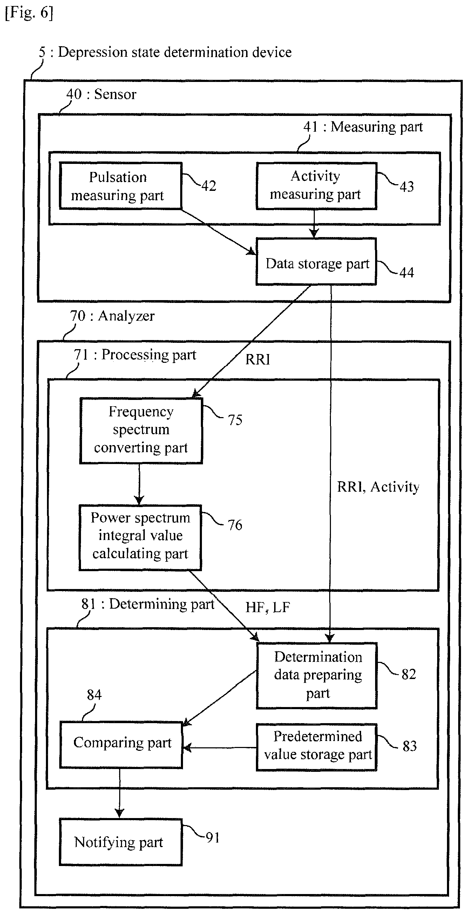

Fig. 6 is a block diagram showing a configuration of a depression state determination device 5 according to a fifth embodiment of the present invention. The depression state determination device 5 shown in Fig. 6 includes a sensor 40 and an analyzer 70. Note that the same components as those of the depression state determination device 1 of the first embodiment are denoted by the same reference signs and a description thereof is omitted.

The sensor 40 is preferably provided with a data storage part 44 that temporarily stores biological information acquired in a measuring part 41 configured by a pulsation measuring part 42 and an activity measuring part 43. Since it is unnecessary that data acquired in the sensor 40 is sequentially transmitted to the analyzer 70 to process the data, an amount of electric power consumed by data communication can be suppressed. For example, it is suitable for a case where a subject performs the measurement, using the sensor at home, and at a later date, a doctor determines whether or not the subject is in the depression state, using the analyzer in a medical institute, or the like.

A processing part 71 of the analyzer 70 reads the data of the RRI stored in the data storage part 44 to perform the frequency spectrum conversion and the power spectrum integral. A determining part 81 performs the determination of the depression state, using the data of the RRI and the activity read from the data storage part 44, and the LF and the HF calculated in a frequency spectrum converting part 75 and a power spectrum integral value calculating part 76. In order to obtain a small, lightweight sensor, a publicly known semiconductor memory is preferably used for the data storage part 44.

(Embodiment 6)

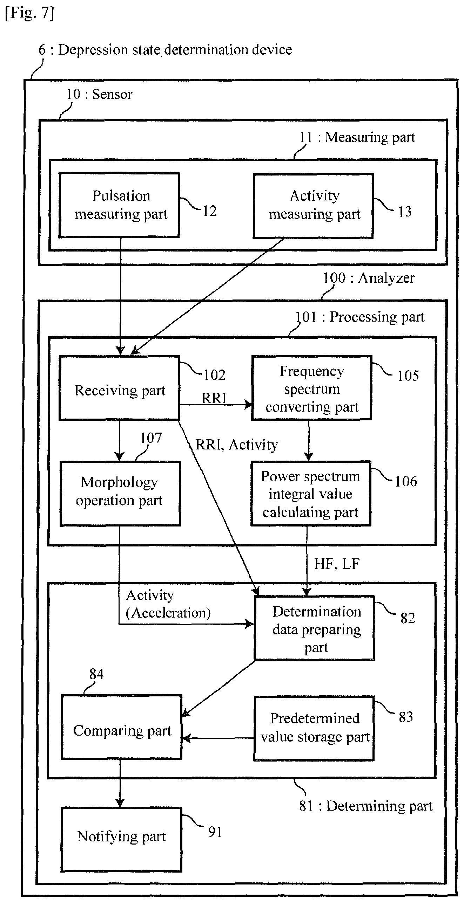

Fig. 7 is a block diagram showing a configuration of a depression state determination device 6 according to a fifth embodiment of the present invention. The depression state determination device 6 shown in Fig. 7 includes a sensor 10 and an analyzer 100. Note that the same components as those of the depression state determination device 1 of the first embodiment are denoted by the same reference signs and a description thereof is omitted.

In the activity measuring part 13 of the measuring part 11 of the sensor 10, any one of the X, Y, Z axes matches the acceleration in the height direction. This is to perform the distinction between the awaking time zone and the sleeping time zone, using the value of the acceleration in the height direction.

The analyzer 100 is provided with a processing part 101 and the determining part 81, and the processing part 101 includes a receiving part 102, a frequency spectrum converting part 105, a power spectrum integral value calculating part 106, and a morphology operation part 107.

The morphology operation part 107 performs a morphology operation to a negative acceleration-time waveform in order to remove noise of the negative acceleration-time waveform output from the receiving part 102. Here, as the morphology operation, as described above, for example, the expansion operation, the contraction operation, the opening processing, and the closing processing, or combinations of these can be applied. Although not shown in Fig. 7, the processing part 101 can also be provided with a binarization processing part that binarizes a magnitude of a value of the negative acceleration with the predetermined value C7 used as a threshold value prior to the processing in the morphology operation part 107. In the binarization processing part, for example, if a negative acceleration T is C7 or more, the negative acceleration is considered to be 0, and if the negative acceleration T is less than C7, it is considered to be 1. In this manner, the binarization processing is performed to the acceleration prior to the morphology operation, which can shorten processing time required for the morphology operation. The value of the predetermined value C7 is not particularly limited, but, for example, is preferably -0.85 g, more preferably, -0.8 g, and still more preferably -0.75 g (here, the unit g represents a magnitude of a gravitational acceleration).

The determining part 81 compares the value of the negative acceleration T processed in the morphology operation part 107 of the processing part 101 with the predetermined value C7, and determines a time zone when the formula (9) is satisfied as the sleeping time zone, and determines a time zone when the formula (9) is not satisfied as the awaking time zone.

$$ T \geq C7 \cdots (9) $$

Here, C7 is a constant number.

Here, a description has been given, using the first distinction method of simply distinguishing the awaking time zone from the sleeping time zone, using the formula (9). However, as described above, the method in which the longest time zone of the time zone when the formula (9) is satisfied is determined as the sleeping time zone, and other than the relevant sleeping time zone is determined as the awaking time zone (the second distinction method), or the method in which the time zone when the formula (9) is satisfied continuously for the predetermined time or longer is determined as the sleeping time zone, and other than the relevant sleeping time zone is determined as the awaking time zone (the third distinction method) can be employed.

This application claims the benefit of the priority date of Japanese patent application No. 2014-172075 filed on August 26, 2014.

(Verification 1)

Verification of usefulness of the depression state determination device of the present invention is performed, using the depression state determination device 1 of the first embodiment. For two days, a small electrocardiograph is attached to each of eight subjects A to H, who are each diagnosed with the depression state by a doctor, and visit a medical institution, to measure the RRI (unit: ms) and the activity (the acceleration) (unit: dimensionless quantity) in the awaking time zone and in the sleeping time zone. Moreover, in parallel to the measurement of the RRI and the activity, medical inquiry of a time-lapse of a symptom of the depression state, a current medication situation of mental illness and the like, a depression state level, symptoms, and a sleeping state is performed. For the depression state level, an evaluation reference in which the depression state is classified into ten stages in accordance with a degree of seriousness (prepared by Fatigue Science Laboratory Inc.) is used. The RRI measured with the electrocardiograph is subjected to the frequency spectrum conversion, and the power spectrum integral is performed to the obtained first power spectrum $F^2$ (unit: $ms^2$/Hz) to thereby calculate the LF and the HF (unit: $ms^2$). The values of the RRI, the activity, the LF and HF/LF are applied to the method for determining the depression state performed by the depression state determination device of the present invention, that is, the formulas (1) to (6) to verify whether or not the respective formulas are satisfied. In the present verification, as to the predetermined values of the formulas (1) to (6), C1 = 170 ms, C2 = 0.09 $ms^2$, C3 = 4, C4 = 1900 ms, C5 = 70$ms^2$, and C6 = 0.005, and as to the integral range of the LF and the HF, Lf1 = 0.04 Hz, Lf2 = 0.15 Hz, Hf1 = 0.15 Hz, and Hf2 = 0.4 Hz. Moreover, the distinction of the awaking time zone and the sleeping time zone is performed, based on the result of the self-report of each of the subjects.

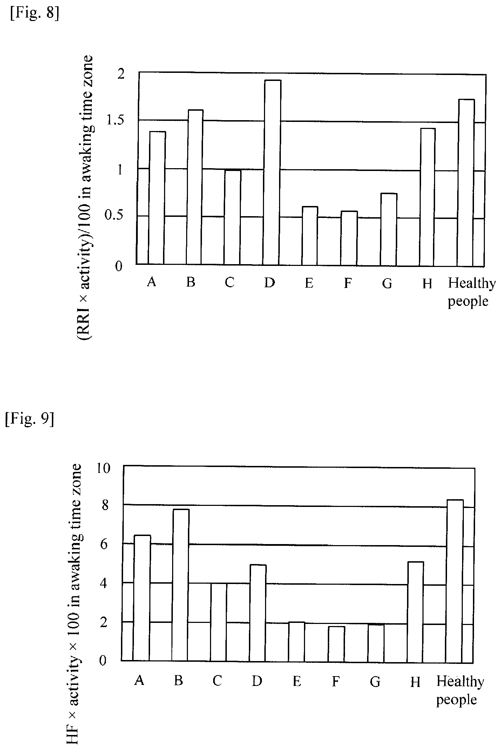

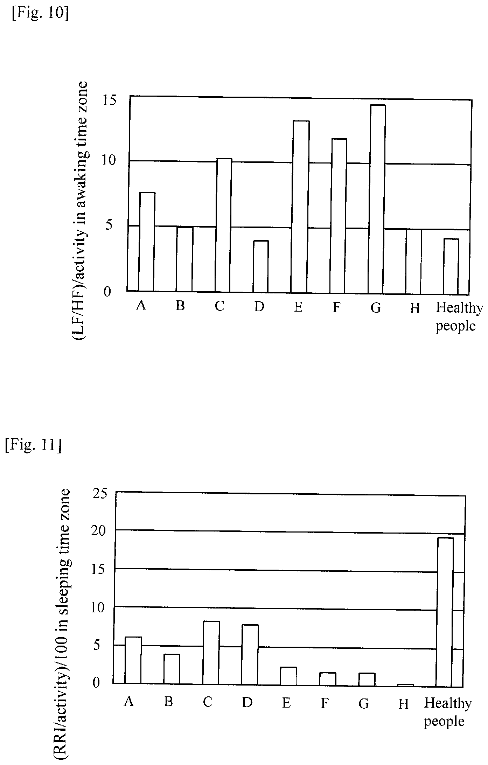

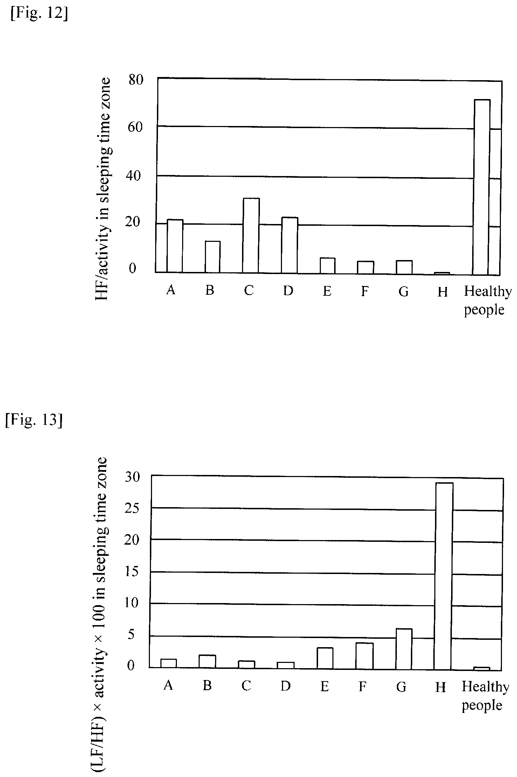

Figs. 8 to 13 show diagrams showing examples in which the formulas (1) to (6) of the method for determining the depression state performed by the depression state determination device of the present invention are applied to the subjects, respectively. A vertical axis in Fig. 8 indicates an (RRI × activity)/100 in the awaking time zone, a vertical axis in Fig. 9 indicates HF × activity × 100 in the awaking time zone, and a vertical axis in Fig. 10 indicates (LF/HF)/activity in the awaking time zone. A vertical axis in Fig. 11 indicates (RRI/activity)/100 in the sleeping time zone, a vertical axis in Fig. 12 indicates HF/activity in the sleeping time zone, and a vertical axis in Fig. 13 indicates (LF/HF) × activity × 100 in the sleeping time zone. A horizontal axis in each of Figs. 8 to 13 indicates the subjects. For reference, a result of an arithmetic average value of eight healthy people is shown in each of Figs. 8 to 13. From Figs. 8 to 13, it can be understood that the values obtained by multiplying/dividing the RRI or the like indicating the autonomic nerve activity by the activity, that is, the values in the left sides of formulas (1) to (6) are different between the healthy people and the patients in the depression state. Especially, in Figs. 11 to 13 showing the results from using measurement data in the sleeping time zone, the difference between the healthy people and the patients in the depression state is remarkable.

Furthermore, as the determination results of the formulas (1) to (6) of the subjects A to H, and the medical inquiry results of the time-lapse of the symptom of the depression state, the current medication situation of mental illness and the like, the depression state level, the symptoms, and the sleeping state are shown in Table 1. As shown in Table 1, when for the subject D, the calculation is performed, using the formulas (1) and (3), the formulas (1) and (3) are not satisfied, so that a determination result that the subject D is not in the depression state is obtained. It is considered that the depression state level of the subject D is 2 (the subject D can live a normal social life, can work, while rest is often needed because of physical fatigue), which is relatively mild, and that no medication affects this state.

On the other hand, in all the cases other than the above-described case, the depression state can be determined. Accordingly, for the eight subjects, the formulas (1) to (6) of the method for determining the depression state performed by the depression state determination device of the present invention are calculated, and as a result, in 46 cases of 48 cases (= 8 subjects × 6 ways), the subjects can be determined to be in the depression state (about 95%).

[Table 1]

| Item | | | Subject | | | | | | | |
|---|---|---|---|---|---|---|---|---|---|---|
| | | | A | B | C | D | E | F | G | H |
| Determination result * 1 | Condition A | (1) Pulsation interval × Activity < C1 | ○ | ○ | ○ | × | ○ | ○ | ○ | ○ |
| | | (2) HF × Activity < C2 | ○ | ○ | ○ | ○ | ○ | ○ | ○ | ○ |
| | | (3) (LF/HF) / Activity > C3 | ○ | ○ | ○ | × | ○ | ○ | ○ | ○ |
| | Condition B | (4) Pulsation interval / Activity < C4 | ○ | ○ | ○ | ○ | ○ | ○ | ○ | ○ |
| | | (5) HF / Activity < C5 | ○ | ○ | ○ | ○ | ○ | ○ | ○ | ○ |
| | | (6) (LF/HF) × Activity > C6 | ○ | ○ | ○ | ○ | ○ | ○ | ○ | ○ |
| | Time-lapse of symptom of depression state | | 1 month | About 2 years | 2 years 3 months | 10 years | - | 2 years 2 months | 1 year 6 months | 4 years 10 months |
| | Current medication situation of mental illness and the like | | With medication | With medication | With medication | No medication | With medication | With medication | With medication | With medication |
| | Depression state level | | 2 | 7 | 5 | 2 | 6 | 8 | 7 | 7 |
| | | Feel languid | ○ | ○ | ○ | × | ○ | ○ | ○ | ○ |
| | | Not recover from fatigue | ○ | ○ | × | × | × | ○ | ○ | ○ |
| | | Cannot sleep | × | ○ | × | × | × | ○ | ○ | ○ |
| | | Poor appetite | ○ | ○ | × | ○ | × | × | × | × |
| | | Have a headache | ○ | ○ | × | × | × | × | ○ | ○ |
| | | Feel heavy in the head | × | × | × | × | × | × | ○ | ○ |

EP 3 187 116 B1

| Item | | | Subject | | | | | | | |
|---|---|---|---|---|---|---|---|---|---|---|
| | | | A | B | C | D | E | F | G | H |
| Medical<br><br>inquiry result | Symptoms*2 | Palpitation | ○ | ○ | × | × | × | ○ | × | × |
| | | Feel a pain in the chest | × | ○ | × | × | × | × | ○ | × |
| | | Dizziness | ○ | ○ | × | × | × | ○ | ○ | ○ |
| | | Feel lightheaded | ○ | ○ | ○ | × | × | ○ | × | × |
| | | Have a rush of blood to the head | × | × | × | × | × | ○ | × | × |
| | | Nausea | × | ○ | × | × | × | × | × | × |
| | | Heavy stomach feeling | × | ○ | ○ | × | × | ○ | × | × |
| | | Feeling of cold | × | × | × | × | ○ | × | × | × |
| | | Diarrhea | × | × | × | × | × | × | ○ | × |
| | | Constipation | ○ | × | ○ | × | ○ | ○ | × | × |
| | | Muscle ache | × | × | × | × | × | ○ | ○ | × |
| | | Have no concentration | ○ | × | ○ | ○ | × | ○ | ○ | ○ |
| | | Have a slight fever | × | ○ | × | × | × | × | × | × |
| | | Loss of physical strength | ○ | ○ | ○ | × | ○ | ○ | ○ | × |
| | | Feel unmotivated | ○ | ○ | ○ | ○ | ○ | ○ | ○ | ○ |

EP 3 187 116 B1

(continued)

| Item | | | Subject | | | | | | | |
|---|---|---|---|---|---|---|---|---|---|---|
| | | | A | B | C | D | E | F | G | H |
| | Sleeping state *2 | Difficulty falling sleep | ○ | × | × | × | × | ○ | × | ○(Panic onset) |
| | | Wake up feeling bad | × | × | × | × | × | ○ | × | × |
| | | Sleepy in the dav time | × | ○ | × | × | ○ | ○ | × | × |
| | | Wake up in the night | × | × | ○(1 time) | ○(3 times) | × | ○(4 times) | × | × |

*1 : Explanatory note of the determination result
○: The determination formula is satisfied (in the depression state). ×: The determination formula is not satisfied (not in the depression state).
*2: Explanatory note of the symptoms and the sleeping state
○: Have the symptom. ×: Have no symptom.

As described above, using the depression state determination device according to the present invention, it can be determined that the subjects are in the depression state with a high probability of about 95%. Thus, the depression state determination device according to the present invention can determine the depression state with ease and with a high accuracy.

(Verification 2)

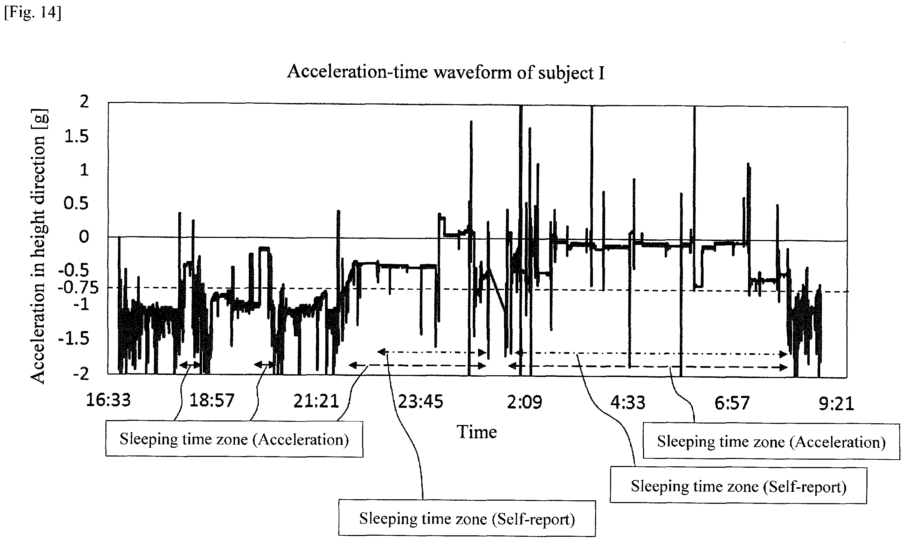

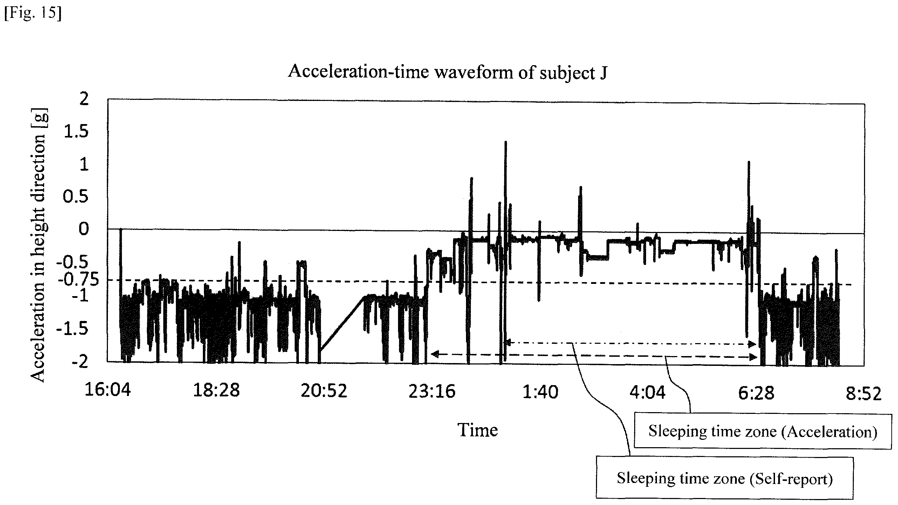

Moreover, applicability of the method in which the awaking time zone and the sleeping time zone are distinguished, using the acceleration of the subjects measured by the depression state determination device according to the present invention is verified. For about 15 hours, a small electrocardiograph is attached to each of seven subjects different from those in the above-described Verification 1, and the acceleration in the height direction of each of the subjects is measured to calculate the negative acceleration T. If the negative acceleration $T \geq C7$, it is determined as the sleeping time zone, and if $T < C7$, it is determined as the awaking time zone. The threshold value $C7$ at this time is set to -0.75 g. As an example, in each of Figs. 14, 15, the negative acceleration-time waveform of each of two subjects (subjects I, J) is shown. In Figs. 14, 15, results of the distinction of the awaking time zone and the sleeping time zone based on the self-report of the subjects are also shown.

The subject I has reported that the sleeping time zones are about 22:46 to 1:18, and about 2:00 to 8:15. In addition, the subject I has reported that about 18:20 to 18:30, and about 20:15 to 20:20, the subject I is not in the sleeping state but in a recumbent state, and that about 0:20 to 1:00, the subject I takes a bath with the electrocardiographic detached, and that other than these time zones is the awaking time zone.

On the other hand, as shown in Fig. 14, since in the distinction by the acceleration, in periods of 18:18 to 18:31, 20:16 to 20:19, 21:51 to 1:18, 1:45 to 8:14, the acceleration exhibits -0.75 g or more, these time zones are determined as the sleeping time zones, and other than the sleeping time zones is determined as the awaking time zone. Moreover, as shown in Fig. 14, in a period of 0:18 to 0:59, the data of the acceleration is lost, and this is attributed to a situation that the electrocardiograph is detached as in the self-report of the subject, and thus, this time zone is determined as neither the sleeping time zone nor the awaking time zone.

The subject J has reported that a period of about 0:03 to 6:10 is the sleeping time zone. In addition, the subject J has reported that in a period of about 20:47 to 21:47, the subject J takes a bath with the electrocardiograph detached, and that other than these time zones is the awaking time zone.

On the other hand, as shown in Fig. 15, since in the distinction by the acceleration, in a time zone of 23:10 to 6:33, the acceleration exhibits roughly -0.75 g or more, this time zone is determined as the sleeping time zone, and other than the sleeping time zone is determined as the awaking time zone. Moreover, as shown in Fig. 15, in a period of 20:47 to 21:47, the data of the acceleration is lost, and this is attributed to a situation that the electrocardiograph is detached as in the self-report of the subject, and thus, this time zone is determined as neither the sleeping time zone nor the awaking time zone.

The distinction result of the awaking time zone and the sleeping time zone based on the acceleration in the height direction, and the distinction result of the awaking time zone and the sleeping time zone based on the self-report of each of the subjects generally coincide with each other, although there is a slight difference. It can be considered that in the case of the distinction of the acceleration, the difference found here is caused because a time zone when the relevant subject is actually awake although he or she takes a recumbent posture is distinguished the sleeping time zone. Although not shown, similar results have been obtained from the five subjects other than the subjects I, J. From the foregoing, when the awaking time zone and the sleeping time zone are distinguished, in place of the method by the self-report, a method by the acceleration in combination of the method by the self-report can also be applied.

REFERENCE SIGNS LIST

| | |
|---|---|
| 1~6: | a depression state determination device |
| 10, 20, 30, 40: | a sensor |
| 11, 21, 31, 41: | a measuring part |
| 12, 22, 32, 42: | a pulsation measuring part |
| 13, 23, 33, 43: | an activity measuring part |
| 24: | an input means |
| 34: | a body temperature measuring part |
| 44: | a data storage part |
| 50, 60, 70, 100: | analyzer |
| 51, 61, 71, 101: | a processing part |

| 52, 62, 102: | a receiving part |
|---|---|
| 55, 65, 75, 105: | a frequency spectrum converting part |
| 56, 66, 76, 106: | a power spectrum integral value calculating part |
| 63: | an abnormal data detecting part |
| 64: | an abnormal data removal part |
| 81: | a determining part |
| 82: | a determination data preparing part |
| 83: | a predetermined value storage part |
| 84: | a comparing part |
| 91: | a notifying part |
| 107: | a morphology operation part |

**Claims**

1. A depression state determination device (1; 2; 3; 4; 5; 6), comprising:

a measuring part (11; 21; 31; 41) adapted for measuring a pulsation interval of a subject, and an acceleration or an angular velocity associated with a movement of the subject (that is hereinafter referred to as an "activity");
a processing part (51; 61; 71; 101) adapted for calculating a value obtained by a procedure including a step of converting the pulsation interval to a frequency spectrum (that is hereinafter referred to as a "power spectrum") and calculating a value of definite integral of a power spectrum from frequency Lf1 to Lf2 (that is hereinafter referred to as an "LF") and a value of definite integral of a power spectrum from frequency Hfl to Hf2 (that is hereinafter referred to as an "HF"); and
a determining part (81) adapted for determining the subject to be a depression state when at least one of the following conditions [A] and [B] is satisfied:

[A] In an awaking time zone of the subject, at least one of the following formulas (1) to (3) is calculated and satisfied:

$$\text{the pulsation interval} \times \text{the activity} < C1 \quad \cdots \quad (1)$$

$$\text{HF} \times \text{the activity} < C2 \quad \cdots \quad (2)$$

$$(\text{LF/HF}) / \text{the activity} > C3 \quad \cdots \quad (3);$$

[B] In a sleeping time zone of the subject, at least one of the following formulas (4) to (6) is calculated and satisfied:

$$\text{the pulsation interval} / \text{the activity} < C4 \quad \cdots \quad (4)$$

$$\text{HF} / \text{the activity} < C5 \quad \cdots \quad (5)$$

$$(\text{LF/HF}) \times \text{the activity} > C6 \quad \cdots \quad (6);$$

wherein
the Hfl > the Lf1, the Hf2 > the Lf2, and the C1 to C6 are constant number.

2. The depression state determination device according to claim 1, wherein
the LF is a value of definite integral of a power spectrum from frequency Lf1 to Lf2, wherein the power spectrum is obtained by converting the pulsation interval to a frequency spectrum and squaring the frequency spectrum, and

the HF is a value of definite integral of a power spectrum from frequency Hfl to Hf2, wherein the power spectrum is obtained by converting the pulsation interval to a frequency spectrum and squaring the frequency spectrum.

3. The depression state determination device according to claim 1 or 2, wherein
the subject is determined to be a depression state when at least one of the formulas is satisfied in the condition [A] and at least one of the formulas is satisfied in the condition [B].

4. The depression state determination device according to claim 1 or 2, wherein
the subject is determined to be a depression state when the formula (3) and at least one of the formulas (1) and (2) are satisfied in the condition [A] and the formula (6) and at least one of the formulas (4) and (5) are satisfied in the condition [B].

5. The depression state determination device according to claim 1 or 2, wherein
the subject is determined to be a depression state when at least one of the formulas is satisfied in the condition [B].

6. The depression state determination device according to any one of claims 1 to 5, wherein
the pulsation interval is an R-R interval between R waves in an electrocardiographic signal.

7. The depression state determination device according to any one of claims 1 to 6, wherein
the awaking time zone and the sleeping time zone is distinguished from each other by measuring an acceleration associated with a posture of the subject and comparing the acceleration with a predetermined value.

8. The depression state determination device according to claim 7, wherein
the acceleration is an acceleration in a height direction associated with a posture of the subject.

9. The depression state determination device according to claim 8, wherein
the sleeping time zone is determined as a time zone which satisfies the following formula (9) and the awaking time zone is determined as a time zone which does not satisfy the following formula (9):

$$\text{a negative acceleration } T \geq C7 \quad \cdots \quad (9),$$

wherein

in the case where the acceleration in the height direction measured in a standing position of the subject is a positive value, the negative acceleration $T$ is calculated by multiplying the acceleration in the height direction by -1,
in the case where the acceleration in the height direction measured in the standing position of the subject is a negative value, the negative acceleration $T$ is the negative value, and
the C7 is a constant number.

10. The depression state determination device according to claim 8, wherein
the sleeping time zone is determined as the longest time zone among a time zone which satisfies the following formula (9) and the awaking time zone is determined as a time zone other than the sleeping time zone:

$$\text{a negative acceleration } T \geq C7 \quad \cdots \quad (9),$$

wherein

in the case where the acceleration in the height direction measured in a standing position of the subject is a positive value, the negative acceleration $T$ is calculated by multiplying the acceleration in the height direction by -1,
in the case where the acceleration in the height direction measured in the standing position of the subject is a negative value, the negative acceleration $T$ is the negative value, and
the C7 is a constant number.

**11.** The depression state determination device according to claim 8, wherein
the sleeping time zone is determined as a time zone which consecutively satisfies the following formula (9) for not less than a predetermined time and the awaking time zone is determined as a time zone other than the sleeping time zone:

$$\text{a negative acceleration } T \geq C7 \quad \cdots \quad (9),$$

wherein

in the case where the acceleration in the height direction measured in a standing position of the subject is a positive value, the negative acceleration T is calculated by multiplying the acceleration in the height direction by -1,
in the case where the acceleration in the height direction measured in the standing position of the subject is a negative value, the negative acceleration T is the negative value, and
the C7 is a constant number.

**12.** The depression state determination device according to any one of claims 9 to 11, wherein
the acceleration is a value after performing a morphology operation to an acceleration-time waveform.

**13.** The depression state determination device according to claim 12, wherein
the morphology operation is at least one of an opening processing and a closing processing performed in a predetermined duration.

**14.** The depression state determination device according to any one of claims 1 to 13, wherein
the subject is determined to be a depression state when the formulas (1) to (6) are satisfied.

**Patentansprüche**

**1.** Vorrichtung (1; 2; 3; 4; 5; 6) zum Bestimmen eines depressiven Zustands, mit:

einem Messteil (11; 21; 31; 41), der dazu eingerichtet ist, ein Pulsintervall eines Probanden und eine Beschleunigung oder eine Winkelgeschwindigkeit, die einer Bewegung des Probanden zugeordnet sind (die nachstehend als "Aktivität" bezeichnet wird), zu messen;
einem Verarbeitungsteil (51; 61; 71; 101), der dazu eingerichtet ist, einen Wert zu berechnen, der durch ein Verfahren erhalten wird, das einen Schritt zum Umwandeln des Pulsintervalls in ein Frequenzspektrum (das nachstehend als "Leistungsspektrum" bezeichnet wird) und zum Berechnen eines Wertes eines bestimmten Integrals eines Leistungsspektrums von einer Frequenz Lf1 bis zu einer Frequenz Lf2 (nachstehend als "LF" bezeichnet) und eines Werts des bestimmten Integrals eines Leistungsspektrums von einer Frequenz Hfl bis zu einer Frequenz Hf2 (nachstehend als "HF" bezeichnet) aufweist; und
einem Bestimmungsteil (81), der dazu eingerichtet ist, zu bestimmen, dass der Proband sich in einem depressiven Zustand befindet, wenn mindestens eine der folgenden Bedingungen [A] und [B] erfüllt ist:

[A] in einem Wachzeitbereich des Probanden wird mindestens eine der folgenden Formeln (1) bis (3) berechnet und erfüllt:

$$\text{Pulsintervall} \times \text{Aktivität} < C1 \qquad (1)$$

$$\text{HF} \times \text{Aktivität} < C2 \qquad (2)$$

$$(\text{LF/HF})/\text{Aktivität} > C3 \qquad (3);$$

[B] in einem Schlafzeitbereich des Probanden wird mindestens eine der folgenden Formeln (4) bis (6)

berechnet und erfüllt:

$$\text{Pulsintervall/Aktivität} < C4 \qquad (4)$$

$$\text{HF/Aktivität} < C5 \qquad (5)$$

$$(\text{LF/HF}) \times \text{Aktivität} > C6 \qquad (6),$$

wobei
Hf1 > Lf1, Hf2 > Lf2 und C1 bis C6 konstante Zahlen sind.

2. Vorrichtung zum Bestimmen eines depressiven Zustands nach Anspruch 1, wobei

LF ein Wert eines bestimmten Integrals eines Leistungsspektrums von der Frequenz Lf1 bis zur Frequenz Lf2 ist, wobei das Leistungsspektrum durch Umwandeln des Pulsintervalls in ein Frequenzspektrum und Quadrieren des Frequenzspektrums erhalten wird, und
HF ein Wert eines bestimmten Integrals eines Leistungsspektrums von der Frequenz Hfl bis zur Frequenz Hf2 ist, wobei das Leistungsspektrum durch Umwandeln des Pulsintervalls in ein Frequenzspektrum und Quadrieren des Frequenzspektrums erhalten wird.

3. Vorrichtung zum Bestimmen eines depressiven Zustands nach Anspruch 1 oder 2, wobei bestimmt wird, dass der Proband sich in einem depressiven Zustand befindet, wenn mindestens eine der Formeln in der Bedingung [A] erfüllt ist und mindestens eine der Formeln in der Bedingung [B] erfüllt ist.

4. Vorrichtung zum Bestimmen eines depressiven Zustands nach Anspruch 1 oder 2, wobei bestimmt wird, dass der Proband sich in einem depressiven Zustand befindet, wenn Formel (3) und mindestens eine der Formeln (1) und (2) in der Bedingung [A] erfüllt sind und die Formel (6) und mindestens eine der Formeln (4) und (5) in der Bedingung [B] erfüllt sind.

5. Vorrichtung zum Bestimmen eines depressiven Zustands nach Anspruch 1 oder 2, wobei bestimmt wird, dass der Proband sich in einem depressiven Zustand befindet, wenn mindestens eine der Formeln in der Bedingung [B] erfüllt ist.

6. Vorrichtung zum Bestimmen eines depressiven Zustands nach einem der Ansprüche 1 bis 5, wobei das Pulsintervall ein R-R-Intervall zwischen R-Wellen in einem elektrokardiographischen Signal ist.

7. Vorrichtung zum Bestimmen eines depressiven Zustands nach einem der Ansprüche 1 bis 6, wobei der Wachzeitbereich und der Schlafzeitbereich voneinander unterschieden werden, indem eine einer Haltung des Probanden zugeordnete Beschleunigung gemessen und die Beschleunigung mit einem vorgegebenen Wert verglichen wird.

8. Vorrichtung zum Bestimmen eines depressiven Zustands nach Anspruch 7, wobei die Beschleunigung eine Beschleunigung in einer Höhenrichtung ist, die einer Haltung des Probanden zugeordnet ist.

9. Vorrichtung zum Bestimmen eines depressiven Zustands nach Anspruch 8, wobei
der Schlafzeitbereich als ein Zeitbereich bestimmt wird, der die folgende Formel (9) erfüllt, und der Wachzeitbereich als ein Zeitbereich bestimmt wird, der die folgende Formel (9) nicht erfüllt:

$$\text{negative Beschleunigung } T \geq C7 \qquad (9),$$

wobei

in dem Fall, in dem die in einer stehenden Position des Probanden gemessene Beschleunigung in der Höhenrichtung ein positiver Wert ist, die negative Beschleunigung T durch Multiplizieren der Beschleunigung in der Höhenrichtung mit -1 berechnet wird,
in dem Fall, in dem die in einer stehenden Position des Probanden gemessene Beschleunigung in der Höhenrichtung ein negativer Wert ist, die negative Beschleunigung T der negative Wert ist, und
C7 eine konstante Zahl ist.

10. Vorrichtung zum Bestimmen eines depressiven Zustands nach Anspruch 8, wobei
der Schlafzeitbereich als der längste Zeitbereich unter einem Zeitbereich bestimmt wird, der die folgende Formel (9) erfüllt, und der Wachzeitbereich als ein Zeitbereich bestimmt wird, der vom Schlafzeitbereich verschieden ist:

$$\text{negative Beschleunigung } T \geq C7 \qquad (9),$$

wobei

in dem Fall, in dem die in einer stehenden Position des Probanden gemessene Beschleunigung in der Höhenrichtung ein positiver Wert ist, die negative Beschleunigung T durch Multiplizieren der Beschleunigung in der Höhenrichtung mit -1 berechnet wird,
in dem Fall, in dem die in einer stehenden Position des Probanden gemessene Beschleunigung in der Höhenrichtung ein negativer Wert ist, die negative Beschleunigung T der negative Wert ist, und
C7 eine konstante Zahl ist.

11. Vorrichtung zum Bestimmen eines depressiven Zustands nach Anspruch 8, wobei
der Schlafzeitbereich als ein Zeitbereich bestimmt wird, der aufeinanderfolgend die folgende Formel (9) für nicht weniger als eine vorgegebene Zeit erfüllt, und der Wachzeitbereich als ein Zeitbereich bestimmt wird, der vom Schlafzeitbereich verschieden ist:

$$\text{negative Beschleunigung } T \geq C7 \qquad (9),$$

wobei

in dem Fall, in dem die in einer stehenden Position des Probanden gemessene Beschleunigung in der Höhenrichtung ein positiver Wert ist, die negative Beschleunigung T durch Multiplizieren der Beschleunigung in der Höhenrichtung mit -1 berechnet wird,
in dem Fall, in dem die in einer stehenden Position des Probanden gemessene Beschleunigung in der Höhenrichtung ein negativer Wert ist, die negative Beschleunigung T der negative Wert ist, und
C7 eine konstante Zahl ist.

12. Vorrichtung zum Bestimmen eines depressiven Zustands nach einem der Ansprüche 9 bis 11, wobei
die Beschleunigung ein Wert ist, nachdem eine Morphologieoperation für eine Beschleunigungszeitwellenform ausgeführt wurde.

13. Vorrichtung zum Bestimmen eines depressiven Zustands nach Anspruch 12, wobei
die Morphologieoperation mindestens eine Operation unter einer Öffnungsverarbeitung und einer Schließverarbeitung ist, die innerhalb einer vorgegebenen Zeitdauer durchgeführt werden.

14. Vorrichtung zum Bestimmen eines depressiven Zustands nach einem der Ansprüche 1 bis 13, wobei
bestimmt wird, dass der Proband sich in einem depressiven Zustand befindet, wenn die Formeln (1) bis (6) erfüllt sind.

## Revendications

1. Dispositif de détermination d'état de dépression (1 ; 2 ; 3 ; 4 ; 5 ; 6), comprenant :

une partie de mesure (11 ; 21 ; 31 ; 41) adaptée pour mesurer un intervalle de pulsations d'un sujet, et une accélération ou une vitesse angulaire associée avec un mouvement du sujet (qui est appelée dans la suite «activité») ;

une partie de traitement (51 ; 61 ; 71 ; 101) adaptée pour calculer une valeur obtenue par une procédure incluant une étape de conversion de l'intervalle de pulsations en un spectre de fréquences (qui est appelé dans la suite "spectre de puissance") et calculer une valeur d'intégrale définie d'un spectre de puissance de la fréquence Lf1 à Lf2 (qui est appelée dans la suite "LF") et une valeur d'intégrale définie d'un spectre de puissance de la fréquence Hfl à Hf2 (qui est appelée dans la suite "HF") ; et

une partie de détermination (81) adaptée pour déterminer que le sujet est un état de dépression lorsqu'au moins l'une des conditions [A] et [B] suivantes est satisfaite :

[A] dans une zone de temps d'éveil du sujet, au moins l'une des formules (1) à (3) suivantes est calculée et satisfaite :

$$\text{l'intervalle de pulsations} \times \text{l'activité} < C1 \quad (1)$$

$$\text{HF} \times \text{l'activité} < C2 \quad (2)$$

$$(\text{LF/HF})/\text{l'activité} > C3 \quad (3) \,;$$

[B] dans une zone de temps de sommeil du sujet, au moins l'une des formules (4) à (6) suivantes est calculée et satisfaite :

$$\text{l'intervalle de pulsations/l'activité} < C4 \quad (4)$$

$$\text{HF/l'activité} < C5 \quad (5)$$

$$(\text{LF/HF}) \times \text{l'activité} > C6 \quad (6) \,;$$

où
la Hf1 > la Lf1, la Hf2 > la Lf2 et les C1 à C6 sont des nombres constants.

2. Dispositif de détermination d'état de dépression selon la revendication 1, dans lequel
la LF est une valeur d'intégrale définie d'un spectre de puissance de la fréquence Lf1 à Lf2, où le spectre de puissance est obtenu en convertissant l'intervalle de pulsations en un spectre de fréquences et élevant au carré le spectre de fréquences, et
la HF est une valeur d'intégrale définie d'un spectre de puissance de la fréquence Hfl à Hf2, où le spectre de puissance est obtenu en convertissant l'intervalle de pulsations en un spectre de fréquences et élevant au carré le spectre de fréquences.

3. Dispositif de détermination d'état de dépression selon la revendication 1 ou 2, dans lequel le sujet est déterminé comme étant dans un état de dépression lorsqu'au moins l'une des formules est satisfaite dans la condition [A] et au moins l'une des formules est satisfaite dans la condition [B].

4. Dispositif de détermination d'état de dépression selon la revendication 1 ou 2, dans lequel
le sujet est déterminé comme étant dans un état de dépression lorsque la formule (3) et au moins l'une des formules (1) et (2) sont satisfaites dans la condition [A] et la formule (6) et au moins l'une des formules (4) et (5) sont satisfaites dans la condition [B].

5. Dispositif de détermination d'état de dépression selon la revendication 1 ou 2, dans lequel
le sujet est déterminé comme étant dans un état de dépression lorsqu'au moins l'une des formules est satisfaite dans la condition [B].

6. Dispositif de détermination d'état de dépression selon l'une quelconque des revendications 1 à 5, dans lequel

l'intervalle de pulsations est un intervalle R-R entre les ondes R dans un signal électrocardiographique.

7. Dispositif de détermination d'état de dépression selon l'une quelconque des revendications 1 à 6, dans lequel la zone de temps d'éveil et la zone de temps de sommeil se distinguent l'une de l'autre en mesurant une accélération associée à une posture du sujet et en comparant l'accélération à une valeur prédéterminée.

8. Dispositif de détermination d'état de dépression selon la revendication 7, dans lequel l'accélération est une accélération dans une direction de la hauteur associée à une posture du sujet.

9. Dispositif de détermination d'état de dépression selon la revendication 8, dans lequel la zone de temps de sommeil est déterminée comme une zone de temps qui satisfait la formule (9) suivante et la zone de temps d'éveil est déterminée comme une zone de temps qui ne satisfait pas la formule (9) suivante :

$$\text{une accélération négative } T \geq C7 \quad (9),$$

où

dans le cas où l'accélération dans la direction de la hauteur mesurée en position debout du sujet est une valeur positive, l'accélération négative T est calculée en multipliant l'accélération dans la direction de la hauteur par -1, dans le cas où l'accélération dans la direction de la hauteur mesurée en position debout du sujet est une valeur négative, l'accélération négative T est la valeur négative et le C7 est un nombre constant.

10. Dispositif de détermination d'état de dépression selon la revendication 8, dans lequel la zone de temps de sommeil est déterminée comme la zone de temps la plus longue parmi une zone de temps qui satisfait la formule (9) suivante et la zone de temps d'éveil est déterminée comme une zone de temps autre que la zone de temps de sommeil :

$$\text{une accélération négative } T \geq C7 \quad (9),$$

où

dans le cas où l'accélération dans la direction de la hauteur mesurée en position debout du sujet est une valeur positive, l'accélération négative T est calculée en multipliant l'accélération dans la direction de la hauteur par -1, dans le cas où l'accélération dans la direction de la hauteur mesurée en position debout du sujet est une valeur négative, l'accélération négative T est la valeur négative, et le C7 est un nombre constant.

11. Dispositif de détermination d'état de dépression selon la revendication 8, dans lequel la zone de temps de sommeil est déterminée comme une zone de temps qui satisfait consécutivement la formule (9) suivante pendant pas moins qu'un temps prédéterminé et la zone de temps d'éveil est déterminée comme une zone de temps autre que la zone de temps de sommeil :

$$\text{une accélération négative } T \geq C7 \quad (9),$$

où

dans le cas où l'accélération dans la direction de la hauteur mesurée en position debout du sujet est une valeur positive, l'accélération négative T est calculée en multipliant l'accélération dans la direction de la hauteur par -1, dans le cas où l'accélération dans la direction de la hauteur mesurée en position debout du sujet est une valeur négative, l'accélération négative T est la valeur négative, et le C7 est un nombre constant.

12. Dispositif de détermination d'état de dépression selon l'une quelconque des revendications 9 à 11, dans lequel

l'accélération est une valeur après exécution d'une opération de morphologie sur une forme d'onde temps-accélération.

13. Dispositif de détermination d'état de dépression selon la revendication 12, dans lequel
l'opération de morphologie est au moins l'un parmi un traitement d'ouverture et un traitement de fermeture exécutés en une durée prédéterminée.

14. Dispositif de détermination d'état de dépression selon l'une quelconque des revendications 1 à 13, dans lequel
le sujet est déterminé comme étant un état de dépression lorsque les formules (1) à (6) sont satisfaites.

[Fig. 1]

[Fig. 2]

1 : Depression state determination device

10 : Sensor

11 : Measuring part

| Pulsation measuring part | 12 | | Activity measuring part | 13 |

50 : Analyzer

51 : Processing part

Receiving part 52

RRI

Frequency spectrum converting part 55

Power spectrum integral value calculating part 56

RRI, Activity

81 : Determining part

HF, LF

Determination data preparing part 82

84

Comparing part

Predetermined value storage part 83

Notifying part 91

[Fig. 3]

2 : Depression state determination device

10 : Sensor

11 : Measuring part

| Pulsation measuring part | ~12 | | Activity measuring part | ~13 |

60 : Analyzer

61 : Processing part

Receiving part ~62 → Abnormal data detecting part ~63

Abnormal data removal part ~64 → RRI → Frequency spectrum converting part ~65

RRI, Activity

Power spectrum integral value calculating part ~66

81 : Determining part

HF, LF

Determination data preparing part ~82

84

Comparing part

Predetermined value storage part ~83

Notifying part ~91

[Fig. 4]

3 : Depression state determination device

[Fig. 5]

4 : Depression state determination device

30 : Sensor

31 : Measuring part

| Pulsation measuring part | ~32 |

| Activity measuring part | ~33 |

| Body temperature measuring part | ~34 |

50 : Analyzer

51 : Processing part

| Receiving part | ~52 |

RRI

| Frequency spectrum converting part | ~55 |

| Power spectrum integral value calculating part | ~56 |

RRI, Activity

81 : Determining part

HF, LF

| Determination data preparing part | ~82 |

84

| Comparing part |

| Predetermined value storage part | ~83 |

| Notifying part | ~91 |

[Fig. 6]

5 : Depression state determination device

40 : Sensor

41 : Measuring part

| Pulsation measuring part | ~42 |

Activity measuring part ~43

Data storage part ~44

70 : Analyzer

71 : Processing part

RRI

Frequency spectrum converting part ~75

RRI, Activity

Power spectrum integral value calculating part ~76

81 : Determining part    HF, LF

Determination data preparing part ~82

84

Comparing part

Predetermined value storage part ~83

Notifying part ~91

[Fig. 7]

6 : Depression state determination device

[Fig. 8]

[Fig. 9]

[Fig. 10]

[Fig. 11]

[Fig. 12]

[Fig. 13]

[Fig. 14]

Acceleration-time waveform of subject I

Sleeping time zone (Acceleration)

Sleeping time zone (Acceleration)

Sleeping time zone (Self-report)

Sleeping time zone (Self-report)

**EP 3 187 116 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2008067892 A
- US 2014058279 A1
- WO 2006056907 A2
- JP 2010179133 A
- JP 2009297474 A
- JP 2014172075 A